# EUROPEAN PATENT SPECIFICATION

(11) **EP 3 145 867 B1**
(45) Date of publication and mention of the grant of the patent: **15.07.2020**
(21) Application number: 15720601.2
(22) Date of filing: 04.05.2015
(51) Int. Cl.: C01F 7/34, C01F 7/44, A61K 8/26, A61Q 1/02, A61Q 19/00, A61K 8/02, C09C 1/00

(54) **ALPHA-AL2O3 FLAKES**
ALPHA-AL2O3-PLÄTTCHEN
FLOCONS DE ALPHA-AL2O3

(30) Priority: 20.05.2014 EP 14001769
(43) Date of publication of application: 29.03.2017
(73) Proprietor: Merck Patent GmbH, 64293 Darmstadt (DE)
(72) Inventor: SUZUKI, Ryuta, Iwaki-City Fukushima-ken 971-8188 (JP); WATANABE, Yukitaka, Iwaki 970-8045 (JP); SCHOEN, Sabine, 45701 Herten (DE); MATSUDA, Noriyuki, Chiba-Shi Chiba 261-0011 (JP)
(86) International application number: PCT/EP2015/000906
(87) International publication number: WO 2015/176796

(56) References cited:
- WO-A1-2008/026829
- US-A- 5 702 519

## Description

The present invention relates to α-Al₂O₃ flakes and the use in variety of applications, especially in cosmetics. Preferably, the present invention relates to functional filler pigments for cosmetics.

Filler pigments for cosmetics have constantly been demanded to have a comfortable and favorable touch and good adherence to skin and last longer (Material Technology (Zairyo Gijutsu), 16 (2), 64 (1998)).

Filler pigments for cosmetics that have transparency serving as a factor of beautiful skin, improve skin tone, and accomplish more natural make-up have been demanded in order to produce healthy beauty (J. Soc. Cosmet. Chem. Jpn. 39 (3), 201-208, (2005)); (HIFU TO BIYO, 124 (4), 4080 (1992)); and (HYOMEN in Japanese), 30(9), 703 (1992)). As optical functions of filler pigments for cosmetics, there have been demanded properties that are capable of covering irregular skin color, and the like, with a thin film and giving a light and natural finish. Moreover, filler pigments for cosmetics are applied to skin and therefore, of course, require a good skin feeling.

Among filler pigments for cosmetics, filler pigments usually having white colors do not influence the final formulation color. The effect that white emulsions with these filler pigments look even whiter, supports positively the precious visual appearance. The filler pigment having white color should offer high chemical and physical stability and is light stable in cosmetic formulations.

The important factors of filler pigments are the particle size, thickness, aspect ratio, shape, surface property, refractive index and the like.

The particle size greatly affects the coloration of the filler pigment because it is closely related with the wavelength of the light. That is, the smaller the particle size, the larger the surface area, thereby increasing the coloration and enhancing reflectivity, and offering a more vivid color.

Suitable filler pigments can be for example Al₂O₃ flakes. Al₂O₃ flakes are well known and can be used as substrate for effect pigments α-Al₂O₃ in the form of hexagonal flakes having a particle diameter which are greater than 10 µm and an aspect ratio (particle diameter/thickness) of 5-10 are known from Japanese Unexamined Patent Application Publication No. 1982-111239.

The Japanese Unexamined Patent Application Publication No. 1991-72572 discloses α-Al₂O₃ in the form of flakes having an average particle diameter of 0.5-3 µm.

The Japanese Unexamined Patent Application Publication No. 1992-39362 describes Al₂O₃ in the form of fine platy particles of a hexagonal crystal system with the plane perpendicular to the c axis grown into a plate.

Al₂O₃ flakes composed of aluminum oxide (as a major constituent) and of titanium dioxide (as a minor constituent) are disclosed in U.S. 5,702,519. The Al₂O₃ flakes are manufactured by using mineralizer which is sulfated alkali metal sulfate such as sodium sulfate or potassium sulfate.

WO 04/60804 A1 discloses Al₂O₃ flakes manufactured by using mineralizer such as metal fluoride. The preferred metal fluorides disclose are sodium fluoride, calcium fluoride, aluminum fluoride and sodium aluminum fluoride. The Al₂O₃ flakes have a particle diameter of 0.1-30 µm, and thickness of 50-200 nm.

On the other hand, the filler pigments for the cosmetics such as a foundation which is directly applied to the skin, those capable of providing a good feeling (skin feeling) are continuously desired (Material Technology (Zairyo Gijutsu), 16 (2), 64 (1998)). As the index to express this skin feeling, there is an average friction coefficient (MIU value) measured by the KES friction tester (KES- SE- DC- tester by KATO TECH. Co., Ltd.).

The average friction coefficient (MIU value) indicates slipperiness of sample, determined as average of µ (a friction coefficient) in a distance of 20 mm. µ (a friction coefficient) is measured to scan surface of the sample by friction block. µ (a friction coefficient) integrates and obtained value divided by 20 mm, and the average friction coefficient (MIU value) is obtained.

In case that the average friction coefficient (MIU value) is smaller, the skin feeling improves because friction becomes less against skin. A MIU value of less than 0.8 is preferred.

The Al₂O₃ flakes of the prior art have the disadvantages that the average friction coefficient (below, it may be abbreviated to MIU value) is relatively high, and the skin feeling is not good and sometimes cosmetic skin has irritation. Therefore, Al₂O₃ flakes are required which do not show the above mentioned advantages and at the same time decrease the MIU value.

In case that Al₂O₃ flakes manufactured by using mineralizer such as fluorine compound, Al₂O₃ flakes are doped by fluorine. But cosmetics including fluorine are not recommended to use in almost countries. Therefore, Al₂O₃ flakes are required which do not contain any fluorine.

The object of the present invention is to provide improved Al₂O₃ flakes having a good skin feeling which do not contain any fluorine or only very small amounts of fluorine and which can be easily prepared and show an excellent skin feeling.

Surprisingly, it has been found that functional filler pigments prepared according to Claim 1 have significantly increased properties with regard to the whiteness, color purity, natural appearance of the skin and the dispersibility into cosmetic formulations.

The Al₂O₃ flakes according to the invention are used, in particular, as filler pigments for cosmetics, especially for the use in decorative and personal care applications. However, they can also be employed in all formulations where alumina flakes are usually employed, such as, for example, in inks, coatings, preferably automotive coatings and plastics.

In a preferred embodiment of the present invention, the Al₂O₃ flakes are prepared starting from an aqueous aluminum salt solution by precipitation with a basic solution. At least one alkali metal sulfate such as sodium or potassium sulfate and at least one dopant such as a titanium compound are added to the starting solution. The precipitation step is followed by drying (evaporation or dehydration by heating), and molten salt treatment including the following steps:
(1) preparation of an aqueous solution of at least one water-soluble and/or insoluble aluminum salt which contains at least one sulfate compound,
(2) adding a basic solution and optionally at least one dopant to the aluminum salt solution (1),
(3) drying of the obtained gel, followed by calcination to obtain Al₂O₃ flakes and alkali salts in a molten salt,
(4) removal of the water soluble parts of the calcined molten salt obtained in step (3).

Examples for aluminum salts can be water-soluble or insoluble salt. Suitable aluminum salts are for example aluminum sulfate, aluminum chloride, aluminum nitrate, poly aluminum chloride, aluminum hydroxide, boehmite, basic aluminum sulfate or combinations thereof. From the view points of the ready availability and handling, aluminum sulfate, aluminum chloride, and aluminum nitrate are preferred.

Examples for a sulfate compound which acts as mineralizer, is for example a metal sulfate. Besides metal sulfates, alkali metal sulfate, alkali earth metal sulfate or combinations thereof are preferred. In particular, an alkali metal sulfate is preferred.

Examples for alkali metal sulfate are sodium sulfate, potassium sulfate, lithium sulfate or combinations thereof. From the view points of the ready availability and low price, sodium sulfate is preferred.

The basic solution which acts as the pH controlling agent for the precipitation is ammonia, sodium hydroxide, potassium hydroxide, sodium carbonate, potassium carbonate or combinations thereof.

From the view points of the ready availability and low price, sodium carbonate and potassium carbonate are more preferable, and sodium carbonate is particularly preferable.

Suitable dopants which could be helpful as control agent for the particle size, thickness, optical properties and/or surface morphology, are preferably selected from the following group of compounds: TiO₂, ZrO₂, SiO₂, In₂O₃, SnO₂, ZnO or combinations thereof. The amount of the dopant is preferably 0.01-5 wt % based on the Al₂O₃ flake.

In a preferred embodiment the dopant is TiO₂, SnO₂ or ZnO. TiO₂ is preferably considered to have a function to suppress the color in the produced Al₂O₃ flakes. ZnO and SnO₂ are preferably considered to promote a decrease in thickness and a growth of the particles and prevent the agglomeration. TiO₂, SnO₂ or ZnO is preferably used in amounts of less than 0.05 wt % based on the Al₂O₃ flakes.

Example for suitable titanium salt for the formation of TiO₂ is titanium tetra-chloride, titanium tri-chloride, titanium oxy sulfate, titanium sulfate or combinations thereof. From the view points of the ready availability and low price, titanium tetra- chloride and titanium sulfate are preferable.

Example for a suitable zinc salt for the formation of ZnO is an acid salt, a halide and an oxide of zinc, specifically zinc sulfate, zinc nitrate and zinc chloride. Example for suitable tin salt for the formation of SnO₂ is an acid salt, a halide and an oxide of tin, specifically tin sulfate, tin nitrate and tin chloride. With regard to the chemical affinity with the aluminum sulfate and superiority in preventing the thickness decrease and agglomeration of flaky crystals, zinc sulfate and tin sulfate are preferred.

The total molar ratio of the sulfate compound to Al₂O₃ is 1 to ≦ 3.5 after step (2).

When the total molar ratio of the sulfate compound to Al₂O₃ is 1 to ≦ 3.5 after step (2), it is considered that the MIU value to be at most 0.8. When the aluminum salt and basic solution undergo the neutralization reaction, molar ratio of the produced alkali metal sulfate to produced Al₂O₃ is 3.0. Therefore, optionally, molar ratio of the sulfate compound to the Al₂O₃ is able to desirably add at most 0.5.

The calcination temperature is from 900 to 1400 °C. Al₂O₃ is transformed from γ-Al₂O₃ to α-Al₂O₃ having a corundum structure by the calcination of at least 900 °C. In case of calcination temperature over 1400 °C, it is increased the probability of damage to equipment. Therefore, the calcination temperature is usually at least 900 °C and more desirably at least 1000 °C, and it is usually at most 1400 °C and more desirably at most 1250 °C.

The Al₂O₃ flakes have a particle size distribution characterized by a Gaussian distribution in which the volume size fractions are distributed as follows:
- D₅₀ is in the range of 5-15 µm, preferably 10-13 µm
- D₈₀ is in the range of less than 20 µm, preferably less than 18 µm.

In this patent application D₅₀ and D₈₀ of the alumina flakes are evaluated by using Malvern MS 2000.

The particle size distribution D₅₀ is also known as the median diameter or the medium value of the particle size distribution, it is the value of the particle diameter at 50 % in the cumulative distribution and is one of the important parameter characterizing the particle size of pigments.

Correspondingly, the D₃₃ value indicates the maximum longitudinal dimensions of the Al₂O₃ flakes, as determined again by means of laser granulometry in the form of sphere equivalents, which 80 % of the particles attain at maximum, or fall below, out of the entirety of all Al₂O₃ particles.

The average thickness is determined on the basis of a cured paint film in which the Al₂O₃ flakes are oriented substantially plane-parallel to the substrate. For this purpose a transverse section of the cured paint film is examined under a scanning electron microscope (SEM), the thickness of 100 Al₂O₃ flakes being ascertained and statistically averaged.

The desired size and thickness distribution can be obtained by suitable classification of the flakes, such as by classifying through selected screens and the like.

The Al₂O₃ flakes have a thickness of ≦ 500 nm.

The Al₂O₃ flakes are α-Al₂O₃ flakes.

In a preferred embodiment the Al₂O₃ flakes have a MIU value of less than 0.8.

The Al₂O₃ flakes according to the present invention are highly suitable as substrate in the preparation of effect pigments. To this end, they are preferably coated with at least one high refractive index layer, like at least one layer of a metal oxide, such as, for example, TiO₂, ZrO₂, SnO₂, ZnO, Ce₂O₃, Fe₂O₃, Fe₃O₄, FeTiO₅, Cr₂O₃, CoO, Co₃O₄, VO₂, V₂O₃, NiO, furthermore of titanium suboxides (TiO₂ partially reduced with oxidation states from < 4 to 2, such as the lower oxides Ti₃O₅, Ti₂O₃, TiO), titanium oxynitrides, FeO(OH), thin semitransparent metal layer, for example comprising Al, Fe, Cr, Ag, Au, Pt or Pd, or combinations thereof. The TiO₂ layer may be in the rutile or anatase modification. In general, the highest quality and gloss and at the same time the most stable effect pigments are obtained when the TiO₂ is in the rutile modification. In order to obtain the rutile modification, an additive can be used which is able to direct the TiO₂ into the rutile modification. Useful rutile directors such as tin dioxide are disclosed in the U.S. 4,038,099 and U.S. 5,433,779 and EP 0 271 767. Preferred effect pigments based on Al₂O₃ flakes are coated with one or more layers of metal oxides, preferably with one metal-oxide layer only, in particular with TiO₂, Fe₂O₃, Fe₃O₄, SnO₂, ZrO₂ or Cr₂O₃. Especially preferred are Al₂O₃ flakes coated with TiO₂ or Fe₂O₃.

The thickness of each high-refractive-index layer depends on the desired interference color. The thickness of each layers on the surface of the Al₂O₃ flakes is preferably 20-400 nm, preferably 30-300 nm, in particular 30-200 nm.

The number of layers on the surface of the Al₂O₃ flakes is preferably one or two, furthermore three, four, five, six or seven layers.

In particular, interference packages consisting of high- and low-refractive-index layers on the surface of the Al₂O₃ flakes result in effect pigments having increased gloss and a further increased interference color or color flop.

Suitable colorless low-refractive-index materials for coating are preferably metal oxides or the corresponding oxide hydrates, such as, for example, SiO₂, Al₂O₃, AIO(OH), B₂O₃, compounds such as MgF₂ or a mixture of the said metal oxides.

In case of multilayer applied on the surface of the Al₂O₃ flakes the interference system is, in particular, a TiO₂-SiO₂-TiO₂ layer sequence.

Furthermore, the effect pigments may also have a semitransparent metal layer as outer layer. Coatings of this type are known, for example, from DE 38 257 02 A1. The metal layers are preferably chromium or aluminum layers having layer thicknesses of 5-25 nm.

Al₂O₃ flakes can also be coated with one or more layers of a metal or metal alloy selected e.g. from chromium, nickel, silver, bismuth, copper, tin, or hastelloy. Al₂O₃ flakes coated with a metal sulfide are coated with sulfides e.g. of tungsten, molybdenum, cerium, lanthanum or rare earth elements.

Furthermore, the effect pigments based on Al₂O₃ flakes can be finally coated with an organic dye as a top coat, preferably with Prussian Blue or Carmine Red.

Particularly preferred effect pigments based on the Al₂O₃ flakes according to the invention have the following layer sequence(s):
Al₂O₃ flake + TiO₂
Al₂O₃ flake + TiO₂/Fe₂O₃
Al₂O₃ flake + Fe₂O₃
Al₂O₃ flake + TiO₂ + Fe₂O₃
Al₂O₃ flake + TiO₂ + Fe₃O₄
Al₂O₃ flake + TiO₂ + SiO₂ + TiO₂
Al₂O₃ flake + Fe₂O₃ + SiO₂ + TiO₂
Al₂O₃ flake + TiO₂/Fe₂O₃ + SiO₂ + TiO₂
Al₂O₃ flake + TiO₂ + SiO₂ + TiO₂/Fe₂O₃
Al₂O₃ flake + TiO₂ + SiO₂
Al₂O₃ flake + TiO₂ + SiO₂/Al₂O₃
Al₂O₃ flake + TiO₂ + Al₂O₃
Al₂O₃ flake + SnO₂
Al₂O₃ flake + SnO₂ + TiO₂
Al₂O₃ flake + SnO₂ + Fe₂O₃
Al₂O₃ flake + SiO₂
Al₂O₃ flake + SiO₂ + TiO₂
Al₂O₃ flake + SiO₂ + TiO₂/Fe₂O₃
Al₂O₃ flake + SiO₂ + Fe₂O₃
Al₂O₃ flake + SiO₂ + TiO₂ + Fe₂O₃
Al₂O₃ flake + SiO₂ + TiO₂ + Fe₃O₄
Al₂O₃ flake + SiO₂ + TiO₂ + SiO₂ + TiO₂
Al₂O₃ flake + SiO₂ + Fe₂O₃ + SiO₂ + TiO₂
Al₂O₃ flake + SiO₂ + TiO₂/Fe₂O₃ + SiO₂ + TiO₂
Al₂O₃ flake + SiO₂ + TiO₂ + SiO₂ + TiO₂/Fe₂O₃
Al₂O₃ flake + SiO₂ + TiO₂ + SiO₂
Al₂O₃ flake + SiO₂ + TiO₂ + SiO₂/Al₂O₃
Al₂O₃ flake + SiO₂ + TiO₂ + Al₂O₃
Al₂O₃ flake + TiO₂ + Prussian Blue
Al₂O₃ flake + TiO₂ + Carmine Red

In this application, the term "coating" or "layer" is taken to mean the complete enveloping of the Al₂O₃ flakes according to the invention.

The effect pigments based on doped or undoped Al₂O₃ flakes preferably consist of 40-90 wt.% of Al₂O₃ flakes and 10-60 wt.% of the coating based on the total pigment.

The Al₂O₃ flakes can be coated by wet chemical coating, by CVD or PVD processes.

The coating of the α- Al₂O₃ flakes with one or more layers, preferably one or more metal oxide layers, is preferably carried out by wet-chemical methods, it being possible to use the wet-chemical coating methods developed for the preparation of pearlescent pigments. Methods of this type are described, for example, in DE 14 67 468, DE 19 59 988, DE 20 09 566, DE 22 14 545, DE 22 15 191, DE 22 44 298, DE 23 13 331, DE 15 22 572, DE 31 37 808, DE 31 37 809, DE 31 51 343, DE 31 51 354, DE 31 51 355, DE 32 11 602, DE 32 35 017 or also in further patent documents and other publications known to the person skilled in the art.

In the case of wet coating, the Al₂O₃ flakes are suspended in water, and one or more hydrolysable metal salts are added at a pH which is suitable for hydrolysis, which is selected in such a way that the metal oxides or metal-oxide hydrates are precipitated directly onto the flakes without secondary precipitations occurring. The pH is usually kept constant by simultaneous metered addition of a base and/or acid. The pigments are subsequently separated off, washed and dried at 50-150 °C for 6-18 h and calcined for 0.5-3 h, where the calcination temperature can be optimized with respect to the respective coating present. In general, the calcination temperatures are 500 - 1000 °C, preferably 600 - 900 °C. If desired, the pigments can be separated off after application of individual coatings, dried and optionally calcined and then re-suspended again for the application of the further layers.

The application of a SiO₂ layer to the Al₂O₃ flake and/or to the already coated Al₂O₃ flake is generally carried out by addition of a potassium or sodium water-glass solution at a suitable pH.

Furthermore, the coating can also be carried out in a fluidized-bed reactor by gas-phase coating, it being possible to use, for example, the methods proposed in EP 0045851 and EP 0106235 for the preparation of pearlescent pigments correspondingly.

The hue and chroma of the effect pigment based on Al₂O₃ flakes according to the invention can be varied in very broad limits through the different choice of the coating amounts or the layer thicknesses resulting there from. Fine tuning for a certain hue and chroma can be achieved beyond the pure choice of amount by approaching the desired color under visual or measurement technology control.

In order to increase the light, water and weather stability, it is frequently advisable, depending on the area of application, to subject the metal oxide coated Al₂O₃ fillers to post-coating or post-treatment. Suitable post-coatings or post-treatments are, for example, the processes described in German Patent 2215191, DE-A 3151354, DE-A 3235017 or DE-A 3334598. This post-coating further increases the chemical and photochemical stability or simplifies the handling of the pigment, in particular the incorporation into various media. In order to improve the weatherability, dispersibility and/or compatibility with the user media, it is possible, for example, for functional coatings of Al₂O₃ or ZrO₂ or mixtures thereof to be applied to the pigment surface. Furthermore, organic post-coatings are possible, for example with silanes, as described, for example, in EP 0090259, EP 0634459, WO 99/57204, WO 96/32446, U.S. 5,759,255, U.S. 5,571,851, WO 01/92425 or in J.J. Ponjeé, Philips Technical Review, Vol. 44, No. 3, 81 ff. and P.H. Harding J.C. Berg, J. Adhesion Sci. Technol. Vol. 11 No. 4, pp. 471-493.

An effect pigment based on Al₂O₃ flakes having the desired size distribution has been found useful in all types of compositions, including plastics, cosmetics, and, in particular in automotive paints.

The Al₂O₃ flakes and the effect pigments based on Al₂O₃ flakes according to the invention are compatible with a multiplicity of color systems, preferably from the area of paints, automotive coatings, industrial coatings, printing inks and cosmetic formulations. For the preparation of printing inks for, for example, gravure printing, flexographic printing, offset printing and offset over varnishing, a multiplicity of binders, in particular water-soluble grades, as sold, for example, by BASF, Marabu, Pröll, Sericol, Hartmann, Gebr. Schmidt, Sicpa, Aarberg, Siegberg, GSB-Wahl, Follmann, Ruco or Coates Screen INKS GmbH, is suitable. The printing inks can be water-based or solvent-based. The Al₂O₃ flakes and the effect pigments according to the invention are furthermore also suitable for the laser marking of paper and plastics and for applications in the agricultural sector, for example for greenhouse sheeting, and, for example, for the coloring of tent awnings.

It goes without saying that, for the various applications, the coated and uncoated Al₂O₃ flakes can also advantageously be used in blends with organic dyes, organic pigments or other pigments, such as, for example, transparent and opaque white, colored and black pigments, and with flake-form iron oxides, organic pigments, holographic pigments, LCPs (liquid crystal polymers) and conventional transparent, colored and black luster pigments based on metal oxide-coated mica and SiO₂ flakes, etc. The pigments according to the invention can be mixed in any ratio with commercially available pigments and fillers.

Fillers which may be mentioned are, for example, natural and synthetic mica, nylon powder, pure or filled melamine resins, talc, SiO₂, glasses, kaolin, oxides or hydroxides of aluminum, magnesium, calcium or zinc, BiOCl, barium sulfate, calcium sulfate, calcium carbonate, magnesium carbonate, carbon, and physical or chemical combinations of these substances. There are no restrictions regarding the particle shape of the filler. It can be, for example, flake-form, spherical or needle-shaped in accordance with requirements.

The Al₂O₃ flakes and the effect pigments based on Al₂O₃ flakes according to the invention are simple and easy to handle. The Al₂O₃ flakes and the effect pigments based on Al₂O₃ flakes can be incorporated into the system in which it is used by simple stirring. Laborious milling and dispersing of the Al₂O₃ flakes and the effect pigments is not necessary.

The Al₂O₃ flakes and the effect pigments based on Al₂O₃ flakes according to the invention can be used for pigmenting coating materials, printing inks, plastics, agricultural films, button pastes, for the coating of seed, for the coloring of food, coatings of medicaments or cosmetic formulations. The concentration of the Al₂O₃ flakes and the effect pigments in the system in which it is to be used for pigmenting is generally between 0.01 and 50 % by weight, preferably between 0.1 and 20 % by weight, based on the overall solids content of the system. This concentration is generally dependent on the specific application.

Plastics containing the Al₂O₃ flakes and the effect pigments based on Al₂O₃ flakes according to the invention in amounts of 0.1 to 50 % by weight, in particular from 0.5 to 7 % by weight, are frequently notable for a particular gloss and shimmer effect.

In the coating sector, especially in automotive coating and automotive finishing, the effect pigments based on Al₂O₃ flakes according to the invention are employed in amounts of 0.5-10 % by weight.

In the coating material, the Al₂O₃ flakes and the effect pigments based on Al₂O₃ flakes according to the invention have the advantage that the desired color and gloss is obtained by a single-layer coating (one-coat systems or as a base coat in a two-coat system).

In the pigmentation of binder systems, for example for paints and printing inks for intaglio, offset or screen printing, the effect pigments based on Al₂O₃ flakes with Stapa®-aluminum and gold bronze pastes from Eckart GmbH have proven particularly suitable. The effect pigment is incorporated into the printing ink in amounts of 2-50 % by weight, preferably 5-30 % by weight and, in particular, 8-15 % by weight. The printing inks containing the effect pigment according to the invention in combination with a metal effect pigment exhibits purer hues and is of improved printability owing to the good viscosity values.

The invention likewise provides pigment preparations containing coated or uncoated Al₂O₃ flakes and further effect pigments, binders and, if desired, additives, the said preparations being in the form of substantially solvent-free, free-flowing granules. Such granules contain up to 95 % by weight of the Al₂O₃ flakes or the effect pigments according to the invention. A pigment preparation in which the effect pigment based on Al₂O₃ flakes of the invention is pasted up with a binder and with water and/or an organic solvent, with or without additives, and the paste is subsequently dried and brought into a compact particulate form, e.g. granules, pellets, briquettes, a master batch or tablets, is particularly suitable as a precursor for printing inks.

The invention thus also relates to the use of the Al₂O₃ flakes in formulations from the areas of paints, coatings, automobile coatings, automotive finishing, industrial coatings, paints, powder coatings, printing inks, security printing inks, plastics, ceramic materials, cosmetics. The Al₂O₃ flakes can furthermore be employed in glasses, in paper, in paper coating, in toners for electrophotographic printing processes, in seed, in greenhouse sheeting and tarpaulins, in thermally conductive, self-supporting, electrically insulating, flexible sheets for the insulation of machines or devices, as absorber in the laser marking of paper and plastics, as absorber in the laser welding of plastics, in pigment pastes with water, organic and/or aqueous solvents, in pigment preparations and dry preparations, such as, for example, granules, for example in clear coats in the industrial and automobile sectors, in sunscreens, as filler, in particular in automobile coatings and automotive finishing.

The invention thus also relates to formulations containing Al₂O₃ flakes and at least one component selected from the group of water, polyols, polar and non-polar oils, fats, waxes, film formers, polymers, copolymers, surfactants, free-radical scavengers, antioxidants, stabilisers, odour enhancers, silicone oils, emulsifiers, solvents, preservatives, thickeners, rheological additives, fragrances, colorants, effect pigments, UV absorbers, surface-active assistants and/or cosmetic active compounds, fillers, binders, pearlescent pigments, color pigments and organic dyes.

All percentage data in this application are per cent by weight, unless indicated otherwise.

The following examples are intended to explain the invention in greater detail, but without restricting it.

### Examples

### Example 1

In 450 ml of deionized water are dissolved 111.9 g of aluminum sulfate 18-hydrate by heating above 60 °C. The resulting solution is designated as the aqueous solution (a).

In 150 ml of deionized water are dissolved 55.0 g of sodium carbonate. The resulting solution is designated as the aqueous solution (b).

The aqueous solution (b) is added with stirring to the aqueous solution (a) kept at about 60 °C. Stirring is continued for 15 minutes. The resulting mixture of the two solutions is a gel. The gel is evaporated to dryness, and the dried product is heated at 1200 °C for 5 hours. Water is added to the heated product to dissolve free sulfate. Insoluble solids are filtered off, washed with water, and finally dried. The obtained alumina flake is examined by X-ray diffractometry. The diffraction pattern has only peaks attributed to corundum structure (α-alumina structure).

D₅₀ is 11.3 µm and D₈₀ is 16.2 µm. The MIU value is 0.55 measured by the KES friction tester.

### Example 2

3.6 g of sodium sulfate is added in the aqueous solution (a) of Example 1. The obtained alumina flake is examined by X-ray diffractometry. The diffraction pattern has only peaks attributed to corundum structure (α-alumina structure).

D₅₀ is 12.3 µm and D₈₀ is 17.0 µm. The MIU value is 0.59 measured by the KES friction tester.

### Example 3

11.9 g of sodium sulfate is added in the aqueous solution (a) of Example 1. The obtained alumina flake is examined by X- ray diffractometry. The diffraction pattern has only peaks attributed to corundum structure (α-alumina structure).

D₅₀ is 12.9 µm and D₈₀ is 17.8 µm. The MIU value is 0.70 measured by the KES friction tester.

### Example 4

0.50 g of 3.44 % solution of titanyl sulfate is added in the aqueous solution (a) of Example 1.

The obtained alumina flake is examined by X-ray diffractometry. The diffraction pattern has only peaks attributed to corundum structure (α-alumina structure).

D₅₀ is 11.5 µm and D₈₀ is 15.8 µm measured by Malvern MS 2000. The MIU value is 0.56 measured by the KES friction tester.

### Comparative Example 1

In 300 ml of deionized water are dissolved 111.9 g of aluminum sulfate 18-hydrate, 57.3 g of anhydride sodium sulfate, and 46.9 g of potassium sulfate by heating above 60 °C. To resulting solution is added 4.06 g of 35.0 % solution of zinc sulfate. The resulting solution is designated as the aqueous solution (a).

In 150 ml of deionized water are dissolved 0.45 g of sodium tertiary phosphate 12-hydrate and 55.0 g of sodium carbonate. The resulting solution is designated as the aqueous solution (b).

The aqueous solution (b) is added with stirring to the aqueous solution (a) kept at about 60 °C. Stirring is continued for 15 minutes. The resulting mixture of the two solutions is a gel. The gel is evaporated to dryness, and the dried product is heated at 1200 °C for 5 hours. Water is added to the heated product to dissolve free sulfate. Insoluble solids are filtered off, washed with water, and finally dried. The obtained alumina flake is examined by X-ray diffractometry. The diffraction pattern has only peaks attributed to corundum structure (α-alumina structure).

D₅₀ is 22.3 µm and D₈₀ is 35.0 µm. The MIU value is 1.00 measured by the KES friction tester.

### Measurements

### Evaluation for particle size D₅₀ and D₈₀

D₅₀ and D₈₀ of the alumina flakes according to the above given (comparative) examples are evaluated by using Malvern MS2000.

### Evaluation for MIU value

MIU value is evaluated by using KES friction tester (KES- SE- DC- tester by KATO TECH. Co., Ltd.).

### Determination of the thickness and particle size and the thickness distribution

0.01 g/l of the alumina flake slurry is prepared and 0.1 ml of this slurry is dropped onto a flat substrate like a silicon wafer. The substrate is dried and cut to adequate size. The substrate is set with almost vertically tilted angle on the base of SEM (Scanning electronic microscope) and the thickness of the alumina flake is determined.

The thickness of more than 100 alumina flakes is measured for the calculation of the thickness distribution. The standard deviation of the thickness is calculated with the Gaussian distribution equation.

Al₂O₃ flakes according to the above given examples are summarized in the following table:

**Table 1:**

| | Added molar ratio of sulfate compound to Al₂O₃ | Total molar ratio of sulfate compound to Al₂O₃ after step (2) | Particles size distribution (µm) | | MIU value |
|---|---|---|---|---|---|
| | | | D₅₀ | D₈₀ | |
| Example 1 | - | 3.0 | 11.3 | 16.2 | 0.55 |
| Example 2 | 0.2 | 3.2 | 12.3 | 17.0 | 0.59 |
| Example 3 | 0.5 | 3.5 | 12.9 | 17.8 | 0.70 |
| Example 4 | - | 3.0 | 11.5 | 15.8 | 0.56 |
| Comparative Example 1 | 4.0 | 7.3 | 22.3 | 35.0 | 1.0 |

### Application examples

**Use example 1: Compact powder**

| **Ingredients** | **Supplier** | **INCI (EU)** | **[%]** |
|---|---|---|---|
| Phase A | | | |
| Pigment of Example 1 | | ALUMINA | 5.00 |
| Xirona® Le Rouge | Merck KGaA | Cl 77491, SILICA | 8.00 |
| Colorona® Bordeaux | Merck KGaA | Cl 77491, MICA | 3.00 |
| RonaFlair® Balance Red | Merck KGaA | Cl 77891, MICA, TIN OXIDE | 3.50 |
| RonaFlair® Balance Blue | Merck KGaA | Cl 77891, MICA, TIN OXIDE | 3.50 |
| Eusolex® T-PRO | Merck KGaA | TITANIUM DIOXIDE, ALUMINA, MANGANESE DIOXIDE | 5.00 |

| Phase B | | | |
|---|---|---|---|
| Crodamol PMP | Croda GmbH | PPG-2 MYRISTYL ETHER PROPIONATE | 29.20 |
| Miglyol 812 N | Sasol Germany GmbH | CAPRYLIC/CAPRIC TRIGLYCERIDE | 15.00 |
| Syncrowax HGLC | Croda GmbH | C18-36 ACID TRIGLYCERIDE | 9.60 |
| Myritol 331 | BASF AG | COCOGLYCERIDES | 9.50 |
| Kester Wachs K 82 P | Koster Keunen Holland BV | SYNTHETIC BEESWAX | 6.00 |
| Antaron V-216 | ISP Global Technologies | PVP/HEXADECENE COPOLYMER | 2.40 |
| Propyl-4-hydroxybenzoate | Merck KGaA | PROPYLPARABEN | 0.10 |

| Phase C | | | |
|---|---|---|---|
| Oxynex® K liquid | Merck KGaA | PEG-8, TOCOPHEROL, ASCORBYL PALMITATE, ASCORBIC ACID, CITRIC ACID | 0.10 |
| RonaCare® Bisabolol | Merck KGaA | BISABOLOL | 0.10 |

Heat phase B to 85 °C and stir until all ingredients are melted. Cool down to 75 °C and add the ingredients of phase A and C while stirring. Fill into godets while compact is still liquid.

It is further possible to use it as creamy eye shadow, as blush for cheeks and color for lips. The filler pigment of Example 1 improves the homogeneous application, supports a smooth and good gliding skin feel and promotes the pearl luster effect.

**Use example 2: Compact powder**

| **Ingredients** | **Supplier** | **INCI (EU)** | **[%]** |
|---|---|---|---|
| Phase A | | | |
| Pigment of Example 2 | | ALUMINA | 5.00 |
| Xirona® Le Rouge | Merck KGaA | Cl 77491, SILICA | 8.00 |
| Colorona® Bordeaux | Merck KGaA | Cl 77491, MICA | 3.00 |
| RonaFlair® Balance Red | Merck KGaA | Cl 77891, MICA, TIN OXIDE | 3.50 |
| RonaFlair® Balance Blue | Merck KGaA | Cl 77891, MICA, TIN OXIDE | 3.50 |
| Eusolex® T-PRO | Merck KGaA | TITANIUM DIOXIDE, ALUMINA, MANGANESE DIOXIDE | 5.00 |

| Phase B | | | |
|---|---|---|---|
| Crodamol PMP | Croda GmbH | PPG-2 MYRISTYL ETHER PROPIONATE | 29.20 |
| Miglyol 812 N | Sasol Germany GmbH | CAPRYLIC/CAPRIC TRIGLYCERIDE | 15.00 |
| Syncrowax HGLC | Croda GmbH | C18-36 ACID TRIGLYCERIDE | 9.60 |
| Myritol 331 | BASF AG | COCOGLYCERIDES | 9.50 |
| Kester Wachs K 82 P | Koster Keunen Holland BV | SYNTHETIC BEESWAX | 6.00 |
| Antaron V-216 | ISP Global Technologies | PVP/HEXADECENE COPOLYMER | 2.40 |
| Propyl-4-hydroxybenzoate | Merck KGaA | PROPYLPARABEN | 0.10 |

| Phase C | | | |
|---|---|---|---|
| Oxynex® K liquid | Merck KGaA | PEG-8, TOCOPHEROL, ASCORBYL PALMITATE, ASCORBIC ACID, CITRIC ACID | 0.10 |
| RonaCare® Bisabolol | Merck KGaA | BISABOLOL | 0.10 |

Heat phase B to 85 °C and stir until all ingredients are melted. Cool down to 75 °C and add the ingredients of phase A and C while stirring. Fill into godets while compact is still liquid.

It is further possible to use it as creamy eye shadow, as blush for cheeks and color for lips. The filler pigment of Example 2 improves the homogeneous application, supports a smooth and good gliding skin feel and promotes the pearl luster effect.

**Use example 3: Compact powder**

| **Ingredients** | **Supplier** | **INCI (EU)** | **[%]** |
|---|---|---|---|
| Phase A | | | |
| Pigment of Example 3 | | ALUMINA | 5.00 |
| Xirona® Le Rouge | Merck KGaA | Cl 77491, SILICA | 8.00 |
| Colorona® Bordeaux | Merck KGaA | Cl 77491, MICA | 3.00 |
| RonaFlair® Balance Red | Merck KGaA | Cl 77891, MICA, TIN OXIDE | 3.50 |
| RonaFlair® Balance Blue | Merck KGaA | Cl 77891, MICA, TIN OXIDE | 3.50 |
| Eusolex® T-PRO | Merck KGaA | TITANIUM DIOXIDE, ALUMINA, MANGANESE DIOXIDE | 5.00 |

| Phase B | | | |
|---|---|---|---|
| Crodamol PMP | Croda GmbH | PPG-2 MYRISTYL ETHER PROPIONATE | 29.20 |
| Miglyol 812 N | Sasol Germany GmbH | CAPRYLIC/CAPRIC TRIGLYCERIDE | 15.00 |
| Syncrowax HGLC | Croda GmbH | C18-36 ACID TRIGLYCERIDE | 9.60 |
| Myritol 331 | BASF AG | COCOGLYCERIDES | 9.50 |
| Kester Wachs K 82 P | Koster Keunen Holland BV | SYNTHETIC BEESWAX | 6.00 |
| Antaron V-216 | ISP Global Technologies | PVP/HEXADECENE COPOLYMER | 2.40 |
| Propyl-4-hydroxybenzoate | Merck KGaA | PROPYLPARABEN | 0.10 |

| Phase C | | | |
|---|---|---|---|
| Oxynex® K liquid | Merck KGaA | PEG-8, TOCOPHEROL, ASCORBYL PALMITATE, ASCORBIC ACID, CITRIC ACID | 0.10 |
| RonaCare® Bisabolol | Merck KGaA | BISABOLOL | 0.10 |

Heat phase B to 85 °C and stir until all ingredients are melted. Cool down to 75 °C and add the ingredients of phase A and C while stirring. Fill into godets while compact is still liquid.

It is further possible to use it as creamy eye shadow, as blush for cheeks and color for lips. The filler pigment of Example 3 improves the homogeneous application, supports a smooth and good gliding skin feel and promotes the pearl luster effect.

**Use example 4: Compact powder**

| **Ingredients** | **Supplier** | **INCI (EU)** | **[%]** |
|---|---|---|---|
| Phase A | | | |
| Pigment of Example 4 | | ALUMINA | 5.00 |
| Xirona® Le Rouge | Merck KGaA | Cl 77491, SILICA | 8.00 |
| Colorona® Bordeaux | Merck KGaA | Cl 77491, MICA | 3.00 |
| RonaFlair® Balance Red | Merck KGaA | Cl 77891, MICA, TIN OXIDE | 3.50 |
| RonaFlair® Balance Blue | Merck KGaA | Cl 77891, MICA, TIN OXIDE | 3.50 |
| Eusolex® T-PRO | Merck KGaA | TITANIUM DIOXIDE, ALUMINA, MANGANESE DIOXIDE | 5.00 |

| Phase B | | | |
|---|---|---|---|
| Crodamol PMP | Croda GmbH | PPG-2 MYRISTYL ETHER PROPIONATE | 29.20 |
| Miglyol 812 N | Sasol Germany GmbH | CAPRYLIC/CAPRIC TRIGLYCERIDE | 15.00 |
| Syncrowax HGLC | Croda GmbH | C18-36ACID TRIGLYCERIDE | 9.60 |
| Myritol 331 | BASF AG | COCOGLYCERIDES | 9.50 |
| Kester Wachs K 82 P | Koster Keunen Holland BV | SYNTHETIC BEESWAX | 6.00 |
| Antaron V-216 | ISP Global Technologies | PVP/HEXADECENE COPOLYMER | 2.40 |
| Propyl-4-hydroxybenzoate | Merck KGaA | PROPYLPARABEN | 0.10 |

| Phase C | | | |
|---|---|---|---|
| Oxynex® K liquid | Merck KGaA | PEG-8, TOCOPHEROL, ASCORBYL PALMITATE, ASCORBIC ACID, CITRIC ACID | 0.10 |
| RonaCare® Bisabolol | Merck KGaA | BISABOLOL | 0.10 |

Heat phase B to 85 °C and stir until all ingredients are melted. Cool down to 75 °C and add the ingredients of phase A and C while stirring. Fill into godets while compact is still liquid.

It is further possible to use it as creamy eye shadow, as blush for cheeks and color for lips. The filler pigment of Example 4 improves the homogeneous application, supports a smooth and good gliding skin feel and promotes the pearl luster effect.

**Use example 5: Eye shadow**

| **Ingredients** | **Supplier** | **INCI (EU)** | **[%]** |
|---|---|---|---|
| Phase A | | | |
| Pigment of Example 1 | | ALUMINA | 13.75 |
| Timiron® Glam Silver | Merck KGaA | ALUMINA, Cl 77891, TIN OXIDE | 5.00 |
| RonaFlair® Extender W | Merck KGaA | MICA, Cl 77891 | 2.50 |
| Timiron® Splendid Violet | Merck KGaA | Cl 77891, MICA, SILICA | 1.25 |

| Phase B | | | |
|---|---|---|---|
| Parteck® LUB Talc | Merck KGaA | TALC | 57.82 |
| Corn Starch | Dr. Hauser GmbH | ZEA MAYS | 8.76 |
| Magnesium Stearate | Merck KGaA | MAGNESIUM STEARATE | 2.92 |
| Unipure Pink LC 583 | S.Goldmann GmbH & Co. KG | Cl 77742 | 3.00 |

| Phase C | | | |
|---|---|---|---|
| Ceraphyl 368 | ISP Global Technologies | ETHYLHEXYL PALMITATE | 4.92 |
| Propyl-4-hydroxybenzoate | Merck KGaA | PROPYLPARABEN | 0.08 |

Mix the ingredients of phase B until the blend is uniform. Add the pigments of phase A. Then add the presolved phase C while stirring strongly. The powder is pressed between 30-40 bar.

The filler pigment of Example 1 is improving the structure of the powder and the pay-off significantly. Furthermore it reduces the forming of greasy spots on the surface of the powder - an effect frequently appearing when using an eye shadow often.

**Use example 6: Eye shadow**

| **Ingredients** | **Supplier** | **INCI (EU)** | **[%]** |
|---|---|---|---|
| Phase A | | | |
| Pigment of Example 2 | | ALUMINA | 13.75 |
| Timiron® Glam Silver | Merck KGaA | ALUMINA, Cl 77891, TIN OXIDE | 5.00 |
| RonaFlair® Extender W | Merck KGaA | MICA, Cl 77891 | 2.50 |
| Timiron® Splendid Violet | Merck KGaA | Cl 77891, MICA, SILICA | 1.25 |

| Phase B | | | |
|---|---|---|---|
| Parteck® LUB Talc | Merck KGaA | TALC | 57.82 |
| Corn Starch | Dr. Hauser GmbH | ZEA MAYS | 8.76 |
| Magnesium Stearate | Merck KGaA | MAGNESIUM STEARATE | 2.92 |
| Unipure Pink LC 583 | S.Goldmann GmbH & Co. KG | Cl 77742 | 3.00 |

| Phase C | | | |
|---|---|---|---|
| Ceraphyl 368 | ISP Global Technologies | ETHYLHEXYL PALMITATE | 4.92 |
| Propyl-4-hydroxybenzoate | Merck KGaA | PROPYLPARABEN | 0.08 |

Mix the ingredients of phase B until the blend is uniform. Add the pigments of phase A. Then add the presolved phase C while stirring strongly. The powder is pressed between 30-40 bar.

The filler pigment of Example 2 is improving the structure of the powder and the pay-off significantly. Furthermore it reduces the forming of greasy spots on the surface of the powder - an effect frequently appearing when using an eye shadow often.

**Use example 7: Eye shadow**

| **Ingredients** | **Supplier** | **INCI (EU)** | **[%]** |
|---|---|---|---|
| Phase A | | | |
| Pigment of Example 3 | | ALUMINA | 13.75 |
| Timiron® Glam Silver | Merck KGaA | ALUMINA, Cl 77891, TIN OXIDE | 5.00 |
| RonaFlair® Extender W | Merck KGaA | MICA, Cl 77891 | 2.50 |
| Timiron® Splendid Violet | Merck KGaA | Cl 77891, MICA, SILICA | 1.25 |

| Phase B | | | |
|---|---|---|---|
| Parteck® LUB Talc | Merck KGaA | TALC | 57.82 |
| Corn Starch | Dr. Hauser GmbH | ZEA MAYS | 8.76 |
| Magnesium Stearate | Merck KGaA | MAGNESIUM STEARATE | 2.92 |
| Unipure Pink LC 583 | S.Goldmann GmbH & Co. KG | Cl 77742 | 3.00 |

| Phase C | | | |
|---|---|---|---|
| Ceraphyl 368 | ISP Global Technologies | ETHYLHEXYL PALMITATE | 4.92 |
| Propyl-4-hydroxybenzoate | Merck KGaA | PROPYLPARABEN | 0.08 |

Mix the ingredients of phase B until the blend is uniform. Add the pigments of phase A. Then add the presolved phase C while stirring strongly. The powder is pressed between 30-40 bar.

The filler pigment of Example 3 is improving the structure of the powder and the pay-off significantly. Furthermore it reduces the forming of greasy spots on the surface of the powder - an effect frequently appearing when using an eye shadow often.

**Use example 8: Eye shadow**

| **Ingredients** | **Supplier** | **INCI (EU)** | **[%]** |
|---|---|---|---|
| Phase A | | | |
| Pigment of Example 4 | | ALUMINA | 13.75 |
| Timiron® Glam Silver | Merck KGaA | ALUMINA, Cl 77891, TIN OXIDE | 5.00 |
| RonaFlair® Extender W | Merck KGaA | MICA, Cl 77891 | 2.50 |
| Timiron® Splendid Violet | Merck KGaA | Cl 77891, MICA, SILICA | 1.25 |

| Phase B | | | |
|---|---|---|---|
| Parteck® LUB Talc | Merck KGaA | TALC | 57.82 |
| Corn Starch | Dr. Hauser GmbH | ZEA MAYS | 8.76 |
| Magnesium Stearate | Merck KGaA | MAGNESIUM STEARATE | 2.92 |
| Unipure Pink LC 583 | S.Goldmann GmbH & Co. KG | Cl 77742 | 3.00 |

| Phase C | | | |
|---|---|---|---|
| Ceraphyl 368 | ISP Global Technologies | ETHYLHEXYL PALMITATE | 4.92 |
| Propyl-4-hydroxybenzoate | Merck KGaA | PROPYLPARABEN | 0.08 |

Mix the ingredients of phase B until the blend is uniform. Add the pigments of phase A. Then add the presolved phase C while stirring strongly. The powder is pressed between 30-40 bar.

The filler pigment of Example 4 is improving the structure of the powder and the pay-off significantly. Furthermore it reduces the forming of greasy spots on the surface of the powder - an effect frequently appearing when using an eye shadow often.

**Use example 9: Cream**

| **Ingredients** | **Supplier** | **INCI (EU)** | **[%]** |
|---|---|---|---|
| Phase A | | | |
| Pigment of Example 1 | | ALUMINA | 2.00 |
| Timiron® Synwhite 40 | Merck KGaA | SYNTHETIC FLUORPHLOGOPITE, Cl 77891 (TITANIUM DIOXIDE), TIN OXIDE | 3.00 |
| Water, demineralized | Merck KGaA | AQUA (WATER) | 62.80 |
| Eusolex® UV-Pearls® OB-S | Merck KGaA | AQUA (WATER), OCTOCRYLENE, SORBITOL, BUTYL METHOXYDIBENZOYLMET HANE, SILICA, PVP, CHLORPHENESIN, PHENOXYETHANOL, DISODIUM EDTA | 15.00 |
| 1.3-Butanediol | Merck KGaA | BUTYLENE GLYCOL | 3.00 |

| Phase B | | | |
|---|---|---|---|
| Miglyol 812 N | Sasol Germany GmbH | CAPRYLIC/CAPRIC TRIGLYCERIDE | 3.00 |
| Cetiol CC | Cognis GmbH | DICAPRYLYL CARBONATE | 2.50 |
| Sepinov P88 | Seppic GmbH | SODIUM ACRYLATE, ACRYLOYL DIMETHYLTAURATE, DIMETHYLACRYLAMIDE CROSSPOLYMER | 2.00 |
| Xiameter® PMX-0345 | Dow Corning | CYCLOPENTASILOXANE, CYCLOHEXASILOXANE | 2.00 |
| Xiameter® PMX-200 (100cs) | Dow Corning | DIMETHICONE | 1.00 |
| RonaCare® AP | Merck KGaA | BIS-ETHYLHEXYL HYDROXYDIMETHOXY BENZYLMALONATE | 0.50 |

| Phase C | | | |
|---|---|---|---|
| RonaCare® Cyclopeptide | Merck KGaA | AQUA (WATER), ALCOHOL DENAT., LECITHIN, ECTOIN, CYCLOTETRAPEPTIDE-24 AMINOCYCLOHEXANE CARBOXYLATE | 2.00 |
| Euxyl PE 9010 | Schülke & Mayr GmbH | PHENOXYETHANOL, ETHYLHEXYL GLYCERIN | 1.00 |
| Fragrance Sensitive Touch | Vögele | PARFUM | 0.20 |

Add phase A slowly with vigorous stirring to phase B. Homogenize. Afterwards add phase C.

The filler pigment of Example 1 adds body and a pleasant skin feeling to this white emulsion. After application it leaves a nice silver shimmer on the skin.

**Use example 10: Cream**

| **Ingredients** | **Supplier** | **INCI (EU)** | **[%]** |
|---|---|---|---|
| Phase A | | | |
| Pigment of Example 2 | | ALUMINA | 2.00 |
| Timiron® Synwhite 40 | Merck KGaA | SYNTHETIC FLUORPHLOGOPITE, Cl 77891 (TITANIUM DIOXIDE), TIN OXIDE | 3.00 |
| Water, demineralized | Merck KGaA | AQUA (WATER) | 62.80 |
| Eusolex® UV-Pearls® OB-S | Merck KGaA | AQUA (WATER), OCTOCRYLENE, SORBITOL, BUTYL METHOXYDIBENZOYLMET HANE, SILICA, PVP, CHLORPHENESIN, PHENOXYETHANOL, DISODIUM EDTA | 15.00 |
| 1.3-Butanediol | Merck KGaA | BUTYLENE GLYCOL | 3.00 |

| Phase B | | | |
|---|---|---|---|
| Miglyol 812 N | Sasol Germany GmbH | CAPRYLIC/CAPRIC TRIGLYCERIDE | 3.00 |
| Cetiol CC | Cognis GmbH | DICAPRYLYL CARBONATE | 2.50 |
| Sepinov P88 | Seppic GmbH | SODIUM ACRYLATE, ACRYLOYL DIMETHYLTAURATE, DIMETHYLACRYLAMIDE CROSSPOLYMER | 2.00 |
| Xiameter® PMX-0345 | Dow Corning | CYCLOPENTASILOXANE, CYCLOHEXASILOXANE | 2.00 |
| Xiameter® PMX-200 (100cs) | Dow Corning | DIMETHICONE | 1.00 |
| RonaCare® AP | Merck KGaA | BIS-ETHYLHEXYL HYDROXYDIMETHOXY BENZYLMALONATE | 0.50 |

| Phase C | | | |
|---|---|---|---|
| RonaCare® Cyclopeptide | Merck KGaA | AQUA (WATER), ALCOHOL DENAT., LECITHIN, ECTOIN, CYCLOTETRAPEPTIDE-24 AMINOCYCLOHEXANE CAR BOXYLATE | 2.00 |
| Euxyl PE 9010 | Schülke & Mayr GmbH | PHENOXYETHANOL, ETHYLHEXYL GLYCERIN | 1.00 |
| Fragrance Sensitive Touch | Vögele | PARFUM | 0.20 |

Add phase A slowly with vigorous stirring to phase B. Homogenize. Afterwards add phase C.

The filler pigment of Example 2 adds body and a pleasant skin feeling to this white emulsion. After application it leaves a nice silver shimmer on the skin.

**Use example 11: Cream**

| **Ingredients** | **Supplier** | **INCI (EU)** | **[%]** |
|---|---|---|---|
| Phase A | | | |
| Pigment of Example 3 | | ALUMINA | 2.00 |
| Timiron® Synwhite 40 | Merck KGaA | SYNTHETIC FLUORPHLOGOPITE, Cl 77891 (TITANIUM DIOXIDE), TIN OXIDE | 3.00 |
| Water, demineralized | Merck KGaA | AQUA (WATER) | 62.80 |
| Eusolex® UV-Pearls® OB-S | Merck KGaA | AQUA (WATER), OCTOCRYLENE, SORBITOL, BUTYL METHOXYDIBENZOYLMET HANE, SILICA, PVP, CHLORPHENESIN, PHENOXYETHANOL, DISODIUM EDTA | 15.00 |
| 1.3-Butanediol | Merck KGaA | BUTYLENE GLYCOL | 3.00 |

| Phase B | | | |
|---|---|---|---|
| Miglyol 812 N | Sasol Germany GmbH | CAPRYLIC/CAPRIC TRIGLYCERIDE | 3.00 |
| Cetiol CC | Cognis GmbH | DICAPRYLYL CARBONATE | 2.50 |
| Sepinov P88 | Seppic GmbH | SODIUM ACRYLATE, ACRYLOYL DIMETHYLTAURATE, DIMETHYLACRYLAMIDE CROSSPOLYMER | 2.00 |
| Xiameter® PMX-0345 | Dow Corning | CYCLOPENTASILOXANE, CYCLOHEXASILOXANE | 2.00 |
| Xiameter® PMX-200 (100cs) | Dow Corning | DIMETHICONE | 1.00 |
| RonaCare® AP | Merck KGaA | BIS-ETHYLHEXYL HYDROXYDIMETHOXY BENZYLMALONATE | 0.50 |

| Phase C | | | |
|---|---|---|---|
| RonaCare® Cyclopeptide | Merck KGaA | AQUA (WATER), ALCOHOL DENAT., LECITHIN, ECTOIN, CYCLOTETRAPEPTIDE-24 AMINOCYCLOHEXANE CARBOXYLATE | 2.00 |
| Euxyl PE 9010 | Schülke & Mayr GmbH | PHENOXYETHANOL, ETHYLHEXYL GLYCERIN | 1.00 |
| Fragrance Sensitive Touch | Vögele | PARFUM | 0.20 |

Add phase A slowly with vigorous stirring to phase B. Homogenize. Afterwards add phase C.

The filler pigment of Example 3 adds body and a pleasant skin feeling to this white emulsion. After application it leaves a nice silver shimmer on the skin.

**Use example 12: Cream**

| **Ingredients** | **Supplier** | **INCI (EU)** | **[%]** |
|---|---|---|---|
| Phase A | | | |
| Pigment of Example 4 | | ALUMINA | 2.00 |
| Timiron® Synwhite 40 | Merck KGaA | SYNTHETIC FLUORPHLOGOPITE, Cl 77891 (TITANIUM DIOXIDE), TIN OXIDE | 3.00 |
| Water, demineralized | Merck KGaA | AQUA (WATER) | 62.80 |
| Eusolex® UV-Pearls® | Merck KGaA | AQUA (WATER), OCTOCRYLENE, SORBITOL, BUTYL METHOXYDIBENZOYLMET HANE, SILICA, PVP, CHLORPHENESIN, PHENOXYETHANOL, DISODIUM EDTA | 15.00 |
| 1.3-Butanediol | Merck KGaA | BUTYLENE GLYCOL | 3.00 |

| Phase B | | | |
|---|---|---|---|
| Miglyol 812 N | Sasol Germany GmbH | CAPRYLIC/CAPRIC TRIGLYCERIDE | 3.00 |
| Cetiol CC | Cognis GmbH | DICAPRYLYL CARBONATE | 2.50 |
| Sepinov P88 | Seppic GmbH | SODIUM ACRYLATE, ACRYLOYL DIMETHYL TAU RATE, DIMETHYLACRYLAMIDE CROSSPOLYMER | 2.00 |
| Xiameter® PMX-0345 | Dow Corning | CYCLOPENTASILOXANE, CYCLOHEXASILOXANE | 2.00 |
| Xiameter® PMX-200 | Dow Corning | DIMETHICONE | 1.00 |
| (100cs) | | | |
| RonaCare® AP | Merck KGaA | BIS-ETHYLHEXYL HYDROXYDIMETHOXY BENZYLMALONATE | 0.50 |

| Phase C | | | |
|---|---|---|---|
| RonaCare® Cyclopeptide | Merck KGaA | AQUA (WATER), ALCOHOL DENAT., LECITHIN, ECTOIN, CYCLOTETRAPEPTIDE-24 AMINOCYCLOHEXANE CARBOXYLATE | 2.00 |
| Euxyl PE 9010 | Schülke & Mayr GmbH | PHENOXYETHANOL, ETHYLHEXYL GLYCERIN | 1.00 |
| Fragrance Sensitive Touch | Vögele | PARFUM | 0.20 |

Add phase A slowly with vigorous stirring to phase B. Homogenize. Afterwards add phase C.

The filler pigment of Example 4 adds body and a pleasant skin feeling to this white emulsion. After application it leaves a nice silver shimmer on the skin.

**Use example 13: Cream**

| **Ingredients** | **Supplier** | **INCI (EU)** | **[%]** |
|---|---|---|---|
| Phase A | | | |
| Xirona® Le Rouge | Merck KGaA | Cl 77491, SILICA | 8.00 |
| Pigment of Example 1 | | ALUMINA | 5.00 |
| Colorona® Bordeaux | Merck KGaA | Cl 77491, MICA | 3.00 |
| RonaFlair® Balance Red | Merck KGaA | Cl 77891, MICA, TIN OXIDE | 3.50 |
| RonaFlair® Balance Blue | Merck KGaA | Cl 77891, MICA, TIN OXIDE | 3.50 |
| Eusolex® T-PRO | Merck KGaA | TITANIUM DIOXIDE, ALUMINA, MANGANESE DIOXIDE | 5.00 |

| Phase B | | | |
|---|---|---|---|
| Crodamol PMP | Croda GmbH | PPG-2 MYRISTYL ETHER PROPIONATE | 29.20 |
| Miglyol 812 N | Sasol Germany GmbH | CAPRYLYLIC/CAPRIC TRIGLYCERIDE | 15.00 |
| Syncrowax HGLC | Croda GmbH | C18-36 ACID TRIGLYCERIDE | 9.60 |
| Myritol 331 | BASF AG | COCOGLYCERIDES | 9.50 |
| Kester Wachs K 82 P | Koster Keunen Holland BV | SYNTHETIC BEESWAX | 6.00 |
| Antaron V-216 | ISP Global Technologies | PVP/HEXADECENE COPOLYMER | 2.40 |
| Propyl-4-hydroxybenzoate Phase C | Merck KGaA | PROPYLPARABEN | 0.10 |
| Oxynex® K liquid | Merck KGaA | PEG-8, TOCOPHEROL, ASCORBYL PALMITATE, ASCORBIC ACID, CITIRC ACID | 0.10 |
| RonaCare® Bisabolol | Merck KGaA | BISABOLOL | 0.10 |

Heat phase B to 85 °C and stir until all ingredients are melted. Cool down to 75°C and add the ingredients of phase A and C while stirring. Fill into godets while the 3-in-1 Compact is still liquid.

**Use example 14: Cream**

| **Ingredients** | **Supplier** | **INCI (EU)** | **[%]** |
|---|---|---|---|
| Phase A | | | |
| Timiron® Synwhite 40 | Merck KGaA | SYNTHETIC FLUORPHLOGOPITE, Cl 77891 (TITANIUM DIOXIDE), TIN OXIDE | 3.00 |
| Pigment of Example 2 | | ALUMINA | 2.00 |
| Water, demineralized | Merck KGaA | AQUA (WATER) | 62.80 |
| Eusolex® UV-Pearls® OB-S | Merck KGaA | AQUA (WATER), OCTOCRYLENE, SORBITOL, BUTYL METHOXYDIBENZOYLMET HANE, SILICA, PVP, CHLORPHENESIN, PHENOXYETHANOL, DISODIUM EDTA | 15.00 |
| 1.3-Butanediol Phase B | Merck KGaA | BUTYLENE GLYCOL | 3.00 |
| Miglyol 812 N | Sasol Germany GmbH | CAPRYLYLIC/CAPRIC TRIGLYCERIDE | 3.00 |
| Cetiol CC | Cognis GmbH | DICAPRYLYL CARBONATE | 2.50 |
| Sepinov P88 | Seppic GmbH | SODIUM ACRYLATE, ACRYLOYL DIMETHYLTAURATE, DI METHYLACRYLAM IDE CROSSPOLYMER | 2.00 |
| Xiameter® PMX-0345 | Dow Corning | CYCLOPENTASILOXANE, CYCLOHEXASILOXANE | 2.00 |
| Xiameter® PMX-200 (100cs) | Dow Corning | DIMETHICONE | 1.00 |
| RonaCare® AP | Merck KGaA | BIS-ETHYLHEXYL HYDROXYDIMETHOXY BENZYLMALONATE | 0.50 |

| Phase C | | | |
|---|---|---|---|
| RonaCare® Cyclopeptide-5 | Merck KGaA | AQUA (WATER), ALCOHOL DENAT., LECITHIN, ECTOIN, CYCLOTETRAPEPTIDE-24 AMINOCYCLOHEXANE CAR BOXYLATE | 2.00 |
| Euxyl PE 9010 | Schülke & Mayr GmbH | PHENOXYETHANOL, ETHYLHEXYL GLYCERIN | 1.00 |
| Fragrance Sensitive Touch | Vögele | PARFUM | 0.20 |

Add phase A slowly with vigorous stirring to phase B. Homogenize. Afterwards add phase C.

**Use example 15: Eyeshadow**

| **Ingredients** | **Supplier** | **INCI (EU)** | **[%]** |
|---|---|---|---|
| Phase A | | | |
| Pigment of Example 3 | | ALUMINA | 13.75 |
| Timiron® Glam Silver | Merck KGaA | ALUMINA, Cl 77891, TIN OXIDE | 5.00 |
| RonaFlair® Extender W | Merck KGaA | MICA, Cl 77891 | 2.50 |
| Timiron® Splendid Violet | Merck KGaA | Cl 77891, MICA, SILICA | 1.25 |
| | | | |

| Phase B | | | |
|---|---|---|---|
| Parteck® LUB Talc | Merck KGaA | TALC | 57.82 |
| Corn Starch | Dr. Hauser GmbH | ZEA MAYS | 8.76 |
| Magnesium Stearate | Merck KGaA | MAGNESIUM STEARATE | 2.92 |
| Unipure Pink LC 583 | S. Goldmann GmbH & Co. KG | Cl 77742 | 3.00 |

| Phase C | | | |
|---|---|---|---|
| Ceraphyl 368 | ISP Global Technologies | ETHYLHEXYL PALMITATE | 4.92 |
| | | | |
| Propyl-4-hydroxybenzoate | Merck KGaA | PROPYLPARABEN | 0.08 |

Mix the ingredients of phase B until the blend is uniform. Add the pigments of phase A. Then add the presolved phase C while stirring strongly. The powder is pressed between 30-40 bar.

**Use example 16: Cream**

| **Ingredients** | **Supplier** | **INCI (EU)** | **[%]** |
|---|---|---|---|
| Phase A | | | |
| RonaFlair® Balance Blue | Merck KGaAs | Cl 77891, MICA, TIN OXIDE | 1.00 |
| RonaFlair® Balance Red | Merck KGaA | Cl 77891, MICA, TIN OXIDE | 1.00 |
| | | | |
| Pigment of Example 1 | | ALUMINA | 1.00 |
| Water, demineralized | Merck KGaA | AQUA | 70.50 |
| Glycerol 85% | Merck KGaA | GLYCERIN, AQUA | 4.00 |
| | | | |
| Hydrolite-5 | Cosnaderm GmbH | PENTYLENE GLYCOL | 2.00 |
| RonaCare® Biotin Plus | Merck KGaA | UREA, DISODIUM PHOSPHATE, BIOTIN, CITRIC | 1.00 |
| D-Panthenol | | ACID PANTHENOL | 0.50 |
| RonaCare® Ectoin | Merck KGaA | ECTOIN | 0.30 |
| Methyl-4-hydroxybenzoate | Merck KGaA | METHYLPARABEN | 0.15 |

| Phase B | | | |
|---|---|---|---|
| Montanov 68 | Seppic GmbH | CETEARYL ALCOHOL, CETEARYL GLUCOSIDE | 4.00 |
| Dow Corning 9040 Silicone Elastomer Blend | Biesterfeld | CYCLOMETHICONE, DIMETHICONE CROSSPOLYMER | 2.50 |
| Tegosoft DEC | Evonik Goldschmidt GmbH | DIETHYLHEXYL CARBONATE | 2.00 |
| Cosmacol ELI | Nordmann, Rassmann GmbH & Co | C12-13 ALKYL LACTATE | 2.00 |
| Arlamol HD | Nordmann, Rassmann GmbH & Co | ISOHEXADECANE | 2.00 |
| Xiameter® PMX-0345 | Dow Corning | CYCLOPENTASILOXANE, CYCLOHEXASILOXANE | 2.00 |
| Span 60 | Croda GmbH | SORBITAN STEARATE | 1.50 |
| | | | |
| Lanette O | BASF AG | CETEARYL ALCOHOL | 1.00 |
| Olive Oil, bio | | OLEA EUROPAEA | 0.50 |
| RonaCare® AP | Merck KGaA | BIS-ETHYLHEXYL HYDROXYDIMETHOXY BENZYLMALONATE | 0.50 |
| Propyl-4-hydroxybenzoate | Merck KGaA | PROPYLPARABEN | 0.05 |

| Phase C | | | |
|---|---|---|---|
| Rhodicare S | Rhodia GmbH | XANTHAN GUM | 0.25 |
| | | | |

| Phase D | | | |
|---|---|---|---|
| Fragrance 46235 Grenadine | Cosnaderm GmbH | PARFUM | 0.20 |

Heat phase A and B separately to 75 °C. Add phase C slowly to phase A while stirring until a homogeneous mixture is obtained. At 75°C add phase B to phase A/C and homogenize for 1 min. (Ultra Turrax T25 at 8000 rpm). Cool down to 35°C and add perfume. Cool down to room temperature while stirring. The pH value should be between 5 - 5.5.

**Use example 17: Cream**

| **Ingredients** | **Supplier** | **INCI (EU)** | **[%]** |
|---|---|---|---|
| Phase A | | | |
| Water, demineralized | Merck KGaA | AQUA | 62.70 |
| 1,2-Propanediol | Merck KGaA | PROPYLENE GLYCOL | 4.00 |
| Chelating Agent | | | 0.10 |
| RonaCare® Ectoin | Merck KGaA | ECTOIN | 1.00 |

| Phase B | | | |
|---|---|---|---|
| RonaFlair® Balance Red | Merck KGaA | Cl 77891, MICA, TIN OXIDE | 3.00 |
| Pigment of Example 4 | | ALUMINA | 2.00 |
| Colorona® Oriental Beige | Merck KGaA | MICA, Cl 77891, Cl 77491 | 1.00 |
| Lexfeel 7 | | NEOPENTYL GLYCOL DIHEPTANOATE | 5.00 |
| Ucon LB 1145 fluid | Mascot Universal Pvt. Ltd. | PPG-33 BUTYL ETHER | 3.00 |
| Tegosoft OER | Evonik Goldschmidt GmbH | OLEYL ERUCATE | 3.00 |
| Talc | | TALC | 2.00 |
| Montanov 202 | Seppic GmbH | ARACHIDYL GLUCOSIDE, ARACHIDYL ALCOHOL, BEHENYL ALCOHOL | 2.00 |
| Eusolex® T-ECO | Merck KGaA | TITANIUM DIOXIDE (NANO), ALUMINA, SIMETHICONE | 2.00 |
| Simulsol 165 | Seppic GmbH | PEG-100 STEARATE, GLYCERYL STEARATE | 5.00 |
| Ginol 16 | Godrej Industries Limited | CETYL ALCOHOL | 1.00 |
| Ginol 18 | Godrej Industries Limited | STEARYL ALCOHOL | 0.50 |
| Hostaphat KL 340 D | Clariant GmbH | TRILAURETH-4 PHOSPHATE | 2.00 |
| Frescolet ML Crystal Phase C | Symrise | MENTHYL LACTATE | 0.05 |
| Phenonip | Clariant GmbH | PHENOXYETHANOL, METHYLPARABEN, ETHYLPARABEN, ISOBUTYLPARABEN, BUTYLPARABEN, PROPYLPARABEN | 0.40 |
| Fragrance (q.s.) | | PARFUM | 0.25 |

Heat phase A and B separately to 80 °C. Ad d phase B slowly to A while stirring. Homogenize. Add the ingredients of phase C to adjust the pH value.

**Use example 18: Cream**

| **Ingredients** | **Supplier** | **INCI (EU)** | **[%]** |
|---|---|---|---|
| Phase A | | | |
| Water, demineralized | Merck KGaA | AQUA | 62.70 |
| Chelating Agent | | | 0.10 |
| 1,2-Propanediol | Merck KGaA | PROPYLENE GLYCOL | 4.00 |
| RonaCare® Ectoin | Merck KGaA | ECTOIN | 1.00 |

| Phase B | | | |
|---|---|---|---|
| RonaFlair® Balance Red | Merck KGaA | Cl 77891, MICA, TIN OXIDE | 3.00 |
| Pigment of Example 3 | | ALUMINA | 2.00 |
| Colorona® Oriental Beige | Merck KGaA | MICA, Cl 77891, Cl 77491 | 1.00 |
| Lexfeel 7 | | NEOPENTYL GLYCOL DIHEPTANOATE | 5.00 |
| Ucon LB 1145 fluid | Mascot Universal Pvt. Ltd. | PPG-33 BUTYL ETHER | 3.00 |
| Tegosoft OER | Evonik Goldschmidt GmbH | OLEYL ERUCATE | 3.00 |
| Talc | | TALC | 2.00 |
| Montanov 202 | Seppic GmbH | ARACHIDYL GLUCOSIDE, ARACHIDYL ALCOHOL, BEHENYL ALCOHOL | 2.00 |
| UV-TITAN M765 | Merck KGaA | TITANIUM DIOXIDE (NANO), TRIETHOXYCAPRYLYLSILA NE, ALUMINA | 2.00 |
| Simulsol 165 | Seppic | PEG-100 STEARATE, GLYCERYL STEARATE | 5.00 |
| Ginol 16 | Godrej Industries Limited | CETYL ALCOHOL | 1.00 |
| Ginol 18 | Godrej Industries Limited | STEARYL ALCOHOL | 0.50 |
| Hostaphat KL 340 D | Clariant GmbH | TRILAURETH-4 PHOSPHATE | 2.00 |
| Frescolet ML Crystal | Symrise | MENTHYL LACTATE | 0.05 |

| Phase C | | | |
|---|---|---|---|
| Phenonip | Clariant GmbH | PHENOXYETHANOL, METHYLPARABEN, ETHYLPARABEN, ISOBUTYLPARABEN, BUTYLPARABEN, PROPYLPARABEN | 0.40 |
| Fragrance (q.s.) | | PARFUM | 0.25 |

Heat phase A and B separately to 80 °C. Add phase B slowly to A while stirring. Homogenize it well. Add the ingredients of phase C at 35 degrees. Neutralize to adjust the pH.

**Use example 19: Cream**

| **Ingredients** | **Supplier** | **INCI (EU)** | **[%]** |
|---|---|---|---|
| Phase A | | | |
| Timiron® BF-1001 | Merck KGaA | MICA, Cl 77891, SILVER OXIDE | 2.00 |
| Colorona® Oriental Beige | Merck KGaA | MICA, Cl 77891, CI 77491 | 0.50 |
| Pigment of Example 1 | | ALUMINA | 0.50 |

| Phase B | | | |
|---|---|---|---|
| Miglyol 812 N | Sasol Germany GmbH | CAPRYLIC/CAPRIC TRIGLYCERIDE | 59.55 |
| Kahlwax 6089 | Kahl & Co. | CERA MICROCRISTALLINA | 27.40 |
| Paraffin viscous | Merck KGaA | PARAFFINUM LIQUIDUM | 10.00 |
| Oxynex® K liquid | Merck KGaA | PEG-8, TOCOPHEROL, ASCORBYL PALMITATE, ASCORBIC ACID, CITRIC ACID | 0.05 |

Heat the ingredients of phase B to 85 °C. Ad d phase A and stir until the melt is homogeneous. Cool down slowly to room temperature while stirring slowly and continuously without high shear forces to reach a smooth, homogeneous product.

**Use example 20: Cream**

| **Ingredients** | **Supplier** | **INCI (EU)** | **[%]** |
|---|---|---|---|
| Phase A | | | |
| Eusolex® UV-Pearls® OB-S | Merck KGaA | AQUA, OCTOCRYLENE, SORBITOL, BUTYL METHOXYDIBENZOYLMET HANE, SILICA, PVP, CHLORPHENESIN, PHENOXYETHANOL, DISODIUM EDTA | 15.00 |
| Pigment of Example 2 | | ALUMINA | 1.00 |
| RonaFlair® Balance Blue | Merck KGaA | Cl 77891, MICA, TIN OXIDE | 1.00 |
| RonaFlair® Balance Red | Merck KGaA | Cl 77891, MICA, TIN OXIDE | 1.00 |
| RonaCare® Ectoin | Merck KGaA | ECTOIN | 0.50 |
| 1.3-Butanediol | Merck KGaA | BUTYLENE GLYCOL | 3.00 |
| Water, demineralized | Merck KGaA | AQUA | 68.00 |

| Phase B | | | |
|---|---|---|---|
| Sepinov P88 | | SODIUM ACRYLATE, ACRYLOYL DIMETHYLTAURATE, DIMETHYLACRYLAMIDE CROSSPOLYMER | 2.00 |
| Miglyol 812 N | Sasol Germany GmbH | CAPRYLIC/CAPRIC TRIGLYCERIDE | 3.00 |
| Cetiol CC | BASF AG | DICAPRYLYL CARBONATE | 2.50 |
| Xiameter® PMX-0345 | Dow Corning | CYCLOPENTASILOXANE, CYCLOHEXASILOXANE | 2.00 |
| Xiameter® PMX-200 (100cs) | Dow Corning | DIMETHICONE | 1.00 |

| Phase C | | | |
|---|---|---|---|
| Preservatives (q.s.) | | | 0.00 |
| Fragrance (q.s.) | | PARFUM | 0.00 |

Mix phase A and add this phase slowly with vigorous stirring to phase B. Homogenize. Finally add phase C under stirring.

**Use example 21: Facepowder**

| **Ingredients** | **Supplier** | **INCI (EU)** | **[%]** |
|---|---|---|---|
| Phase A | | | |
| Pigment of Example 1 | | ALUMINA | 15.00 |
| RonaFlair® Silk Mica | Merck KGaA | MICA | 15.00 |
| Eusolex® T-S | Merck KGaA | TITANIUM DIOXIDE (NANO), ALUMINA, STEARIC ACID | 5.00 |
| Titanium -(IV)- oxid | Merck KGaA | TITANIUM DIOXIDE, TITANIUM DIOXIDE (NANO) | 3.00 |
| Unipure Yellow LC 181 | S. Goldmann GmbH & Co. KG | Cl 77492 | 0.30 |
| Unipure Red LC 381 | S. Goldmann GmbH & Co. KG | Cl 77491 | 0.04 |
| Unipure Brown LC 889 | S. Goldmann GmbH & Co. KG | Cl 77491, Cl 77499 | 0.06 |
| Parteck® LUB Talc | Merck KGaA | TALC | 55.20 |
| Parteck® LUB MST | Merck KGaA | MAGNESIUM STEARATE | 2.00 |

| Phase B | | | |
|---|---|---|---|
| Eutanol G | BASF AG | OCTYLDODECANOL | 3.70 |
| all-rac-alpha-Tocopheryl acetate | Merck KGaA | TOCOPHERYL ACETATE | 0.30 |
| Fragrance 200 529 | Fragrance Resources | PARFUM | 0.30 |
| Propyl-4-hydroxybenzoate | Merck KGaA | PROPYLPARABEN | 0.10 |

Grind the ingredients of phase A until the blend is homogeneous. Then add the previously dissolved phase B and grind again until the whole phase A/B is homogeneous. Fill the bulk into pans and press with the desired pressure. The pressure for pans with 36 mm in diameter is approx. 25-35 bar.

**Use example 22: Nail Polish**

| **Ingredients** | **Supplier** | **INCI (EU)** | **[%]** |
|---|---|---|---|
| Xirona® Golden Sky | Merck KGaA | SILICA, Cl 77891, TIN OXIDE | 2.00 |
| Pigment of Example 2 | | ALUMINA | 1.00 |
| RonaFlair® Extender W | Merck KGaA | MICA, Cl 77891 | 1.00 |
| Colorona® Mica Black | Merck KGaA | Cl 77499, MICA, Cl 77891 | 0.15 |
| Coloring Base Ref. 696 | Durlin/France | ETHYL ACETATE, BUTYL ACETATE, NITROCELLULOSE, Cl 47005, PHTHALIC ANHYDRIDE/TRIMELLITIC ANHYDRIDE/GLYCOLS COPOLYMER, ACETYL TRIBUTYL CITRATE, ISOPROPYL ALCOHOL, STEARALKONIUM HECTORITE | 0.40 |
| Coloring Base Ref. 695 | Durlin/France | ETHYL ACETATE, BUTYL ACETATE, NITROCELLULOSE, PHTHALIC ANHYDRIDE/TRIMELLITIC ANHYDRIDE/GLYCOLS COPOLYMER, Cl 42090, ACETYL TRIBUTYL CITRATE, ISOPROPYL ALCOHOL, STEARALKONIUM HECTORITE | 0.20 |
| Thixotropic Nail Lacquer Base 155 | Durlin/France | BUTYL ACETATE, ETHYL ACETATE, NITROCELLULOSE, ACETYL TRIBUTYL CITRATE, PHTHALIC ANHYDRIDE/TRIMELLITIC ANHYDRIDE/GLYCOLS COPOLYMER, ISOPROPYL ALCOHOL, STEARALKONIUM HECTORITE, ADIPIC ACID/FUMARIC ACID/PHTHALIC ACID/TRICYCLODECANE DIMETHANOL COPOLYMER, CITRIC ACID | 95.25 |

Mix all ingredients by stirring at 1000 rpm for 10 min. Avoid introducing air.

**Use example 23: Nude Lipstick**

| **Ingredients** | **Supplier** | **INCI (EU)** | **[%]** |
|---|---|---|---|
| Phase A | | | |
| Timiron® SynBeam Red | Merck KGaA | SYNTHETIC FLUORPHLOGOPITE, Cl 77891, TIN OXIDE | 5.00 |
| Pigment of Example 1 | | ALUMINA | 5.00 |
| Colorona® SynBerry Pink | Merck KGaA | SYNTHETIC FLUORPHLOGOPITE, Cl 77891, Cl 75470, TIN OXIDE | 0.10 |

| Phase B | | | |
|---|---|---|---|
| Oxynex® K liquid | Merck KGaA | PEG-8, TOCOPHEROL, ASCORBYL PALMITATE, ASCORBIC ACID, CITRIC ACID | 0.05 |
| Oxynex® ST Liquid | Merck KGaA | DIETHYLHEXYL SYRINGYLIDENE MALONATE, CAPRYLIC/CAPRIC TRIGLYCERIDE | 0.10 |
| Sensiva PA 20 | Schülke & Mayr GmbH | PHENETHYL ALCOHOL, ETHYLHEXYL GLYCERIN | 1.00 |
| Paraffin viscous | Merck KGaA | PARAFFINUM LIQUIDUM | 2.10 |
| Adeps Lanae | Henry Lamotte Oils GmbH | LANOLIN | 3.50 |
| Paracera C 44 | Azelis Germany GmbH | COPERNICIA CERIFERA, CERESIN | 5.25 |
| Isopropyl Myristate | BASF AG | ISOPROPYL MYRISTATE | 5.60 |
| Wax white | Merck KGaA | CERA ALBA | 8.75 |
| Castor Oil | Henry Lamotte Oils GmbH | RICINUS COMMUNIS (CASTOR) SEED OIL | 63.35 |

| Phase C | | | |
|---|---|---|---|
| Fragrance Pearl | Cosnaderm GmbH | PARFUM | 0.20 |

Heat the ingredients of phase B to 75 °C. Ad d phase A and stir until the melt is homogeneous. Transfer the mixture into a moulding machine, which is heated up to 65 °C, add the perfume and stir about 15 minutes. Fill into a lipstick mould which has been preheated to about 55 °C. Cool down the mould and transfer the cold bullets to mechanisms. A highly glossy appearance can be obtained by subsequent flaming if desired.

**Use example 24: Cream**

| **Ingredients** | **Supplier** | **INCI (EU)** | **[%]** |
|---|---|---|---|
| Phase A | | | |
| RonaCare® Allantoin | Merck KGaA | ALLANTOIN | 0.00 |
| RonaCare® Ectoin | Merck KGaA | ECTOIN | 0.00 |
| Eusolex® UV-Pearls® 2292 | Merck KGaA | AQUA, ETHYLHEXYL METHOXYCINNAMATE, SILICA, PVP, CHLORPHENESIN, DISODIUM EDTA, BHT | 0.02 |
| Eusolex® UV-Pearls® OB-S | Merck KGaA | AQUA, OCTOCRYLENE, SORBITOL, BUTYL METHOXYDIBENZOYLMET HANE, SILICA, PVP, CHLORPHENESIN, PHENOXYETHANOL, DISODIUM EDTA | 0.02 |
| Water, demineralized | Merck KGaA | AQUA | 0.04 |
| Carbopol Ultrez 21 | Gattefossé | ACRYLATES/C10-30 ALKYL | 0.00 |
| | GmbH | ACRYLATE CROSSPOLYMER | |
| Glycerol 85% | Merck KGaA | GLYCERIN, AQUA | 0.01 |
| Rhodicare S | C. H. Erbslöh | XANTHAN GUM | 0.00 |
| Titriplex® III | Merck KGaA | DISODIUM EDTA | 0.00 |

| Phase B | | | |
|---|---|---|---|
| RonaCare® AP | Merck KGaA | BIS-ETHYLHEXYL HYDROXYDIMETHOXY BENZYLMALONATE | 0.00 |
| RonaCare® Bisabolol | Merck KGaA | BISABOLOL | 0.00 |
| Olivem 1000 | Erbslöh | CETEARYL OLIVATE, SORBITAN OLIVATE | 0.00 |
| Isostearyl Isostearate | Gattefossé GmbH | ISOSTEARYL ISOSTEARATE | 0.01 |
| Organic sweet almond oil | IES Labo | PRUNUS AMYGDALUS DULCIS OIL | 0.01 |

| Phase C | | | |
|---|---|---|---|
| Sodium Hydroxide, 10% | Merck KGaA | AQUA, SODIUM HYDROXIDE | 0.00 |

| Phase D | | | |
|---|---|---|---|
| Pigment of Example 3 | | ALUMINA | 0.00 |
| RonaFlair® Balance Green Phase E | Merck KGaA | Cl 77891, MICA, TIN OXIDE | 0.00 |
| Microcare PM2 | Thor Personnal Care SAS | PHENOXYETHANOL, ETHYLPARABEN, METHYLPARABEN | 0.00 |

Disperse Carbopol ultrez 21 in the water. Add the xanthane gum premixed with glycerin. Add the active ingredients and the titriplex. Add the UV-pearls. Prepare phase B and heat A and B up 80°C. Emulsion B in A. Neutralise with C. At temperature < 60 °C add D. At temperature < 30 °C add E.

**Use example 25: Cream**

| **Ingredients** | **Supplier** | **INCI (EU)** | **[%]** |
|---|---|---|---|
| Phase A | | | |
| Tego Care 450 | Evonik Goldschmidt GmbH | POLYGLYCERYL-3 METHYLGLUCOSE DISTEARATE | 3.00 |
| Tegin M | Evonik Goldschmidt GmbH | GLYCERYL STEARATE | 2.00 |
| Lanette 18 | BASF AG | STEARYL ALCOHOL | 2.00 |
| Miglyol 812 | Sasol Germany GmbH | CAPRYLIC/CAPRIC TRIGLYCERIDE | 7.50 |
| Avocado Oil | Gustav Heess GmbH | PERSEA GRATISSIMA OIL | 2.00 |
| Arlamol HD | Croda GmbH | ISOHEXADECANE | 9.50 |

| Phase B | | | |
|---|---|---|---|
| Water, demineralized | Merck KGaA | AQUA | 64.50 |
| Glycerol 85% | Merck KGaA | GLYCERIN, AQUA | 3.00 |
| Pigment of Example 1 | | ALUMINA | 2.50 |

| Phase C | | | |
|---|---|---|---|
| RonaCare® Cyclopeptide-5 | Merck KGaA | AQUA, ALCOHOL, LECITHIN, ECTOIN, CYCLOTETRAPEPTIDE-24 AMINOCYCLOHEXANE CARBOXYLATE | 4.00 |
| Preservatives (q.s.) | | | 0.00 |
| Fragrance (q.s.) | | PARFUM | 0.00 |

Heat phase A and phase B separately to 75° C. Incorporate phase A into phase B while stirring and homogenize. Cool down to 30° C while stirring and add ingredients of phase C.

**Use example 26: Eyeshadow Gel**

| **Ingredients** | **Supplier** | **INCI (EU)** | **[%]** |
|---|---|---|---|
| Phase A | | | |
| Colorona® Precious Gold | Merck KGaA | MICA, Cl 77891, SILICA, Cl 77491, TIN OXIDE | 9.70 |
| Ronastar® Golden Jewel | Merck KGaA | CALCIUM ALUMINUM BOROSILICATE, Cl 77891, SILICA, Cl 77491, TIN OXIDE | 2.00 |
| Colorona® Mica Black | Merck KGaA | Cl 77499, MICA, Cl 77891 | 1.00 |
| Pigment of Example 1 | | ALUMINA | 3.00 |
| Carbopol Ultrez 21 | Gattefossé GmbH | ACRYLATES/C10-30 ALKYL ACRYLATE CROSSPOLYMER | 0.30 |
| Citric Acid Monohydrate | Merck KGaA | CITRIC ACID | 0.00 |
| Water, demineralized | Merck KGaA | AQUA | 63.20 |

| Phase B | | | |
|---|---|---|---|
| RonaCare® Allantoin | Merck KGaA | ALLANTOIN | 0.10 |
| RonaCare® Triethanolamine Preservatives (q.s.) | Merck KGaA | TRIETHANOLAMINE | 0.70 |
| Glycerol, anhydrous | Merck KGaA | GLYCERIN | 2.00 |
| Water, demineralized | Merck KGaA | AQUA | 13.00 |

| Phase C | | | |
|---|---|---|---|
| Lubrajel DV | Azelis Germany GmbH | PROPYLENE GLYCOL, POLYGLYCERYLMETHACR YLATE | 5.00 |

Disperse all pigments and the filler in the water of phase A. Add some drops of citric acid solution to lower the viscosity if necessary, then add the Carbopol Ultrez 21 while stirring. Mix with high agitation until thoroughly dispersed. Mix the ingredients of phase B until a complete solution is obtained. Add phase B slowly to phase A while stirring (not homogenizing), then add phase C while stirring and adjust pH to 7.0 - 7.5 with citric acid solution, if necessary.

**Use example 27: Lipstick**

| **Ingredients** | **Supplier** | **INCI (EU)** | **[%]** |
|---|---|---|---|
| Phase A | | | |
| Timiron® Splendid Gold | Merck KGaA | Cl 77891, MICA, SILICA | 8.00 |
| Colorona® Magenta | Merck KGaA | MICA, Cl 77891, Cl 75470 | 2.50 |
| RonaFlair® Balance Gold | Merck KGaA, Darmstadt, Germany / EMD Chemicals | Cl 77891, MICA, TIN OXIDE | 2.00 |
| Pigment of Example 1 | | ALUMINA | 2.00 |
| Colorona® Oriental Beige | Merck KGaA | MICA, Cl 77891, Cl 77491 | 0.50 |

| Phase B | | | |
|---|---|---|---|
| Castor Oil | Henry Lamotte Oils GmbH | RICINUS COMMUNIS (CASTOR) SEED OIL | 59.45 |
| Wax white | Merck KGaA | CERA ALBA | 8.75 |
| Paracera C 44 | Azelis Germany GmbH | COPERNICIA CERIFERA, CERESIN | 5.25 |
| Adeps Lanae | Henry Lamotte Oils GmbH | LANOLIN | 3.50 |
| Isopropyl Myristate | BASF AG | ISOPROPYL MYRISTATE | 5.60 |
| Paraffin viscous | Merck KGaA | PARAFFINUM LIQUIDUM | 2.10 |
| Propyl-4-hydroxybenzoate | Merck KGaA | PROPYLPARABEN | 0.10 |
| Oxynex® K liquid | Merck KGaA | PEG-8, TOCOPHEROL, ASCORBYL PALMITATE, ASCORBIC ACID, CITRIC ACID | 0.05 |

| Phase C | | | |
|---|---|---|---|
| Fragrance Pearl FC10599 | Cosnaderm GmbH | PARFUM | 0.20 |

Heat the ingredients of phase B to 75 °C. Ad d phase A and stir until the melt is homogeneous. Transfer the mixture into a moulding machine, which is heated up to 65 °C, add the perfume and stir about 15 minutes. Fill into a lipstick mould which has been preheated to about 55 °C. Cool down the mould and transfer the cold bullets to mechanisms. A highly glossy appearance can be obtained by subsequent flaming if desired.

**Use example 28: Loose Powder Eye Shadow**

| **Ingredients** | **Supplier** | **INCI (EU)** | **[%]** |
|---|---|---|---|
| Phase A | | | |
| Colorona® Imperial Topaz | Merck KGaA | MICA, Cl 77163, CI 77491, Cl 77492, Cl 77499 | 30.00 |
| Supra H | Luzenac America | | 27.80 |
| | | | |
| Dry Flo PC | Azelis Germany GmbH | ALUMINUM STARCH OCTENYLSUCCINATE | 16.00 |
| Pigment of Example 1 | | ALUMINA | 12.00 |
| Timiron® Ice Crystal | Merck KGaA | SYNTHETIC FLUORPHLOGOPITE, Cl 77891, TIN OXIDE | 3.00 |
| Kaolin | Merck KGaA | KAOLIN | 4.00 |
| Magnesium Stearate | Whittaker, Clark & Daniels | MAGNESIUM STEARATE | 2.00 |
| Propylparaben | Sutton | PROPYLPARABEN | 0.20 |

| Phase B | | | |
|---|---|---|---|
| Eutanol G | BASF AG | OCTYLDODECANOL | 5.00 |

Combine Phase A with gentle agitation. Spray Phase B onto batch while agitating bulk. Pass entire batch through a jump gap.

**Use example 29: O/W soft face cream**

| **Ingredients** | **Supplier** | **INCI (EU)** | **[%]** |
|---|---|---|---|
| Phase A | | | |
| RonaCare® Pristine Bright™ | Merck KGaA | METHOXYPHENYL T-BUTYLPHENYL PROPANEDIOL | 2.00 |
| RonaCare® AP | Merck KGaA | BIS-ETHYLHEXYL HYDROXYDIMETHOXY BENZYLMALONATE | 1.00 |
| | | | |
| Montanov 202 | Seppic GmbH | ARACHIDYL ALCOHOL, BEHENYL ALCOHOL, | |
| Tegosoft DEC | Evonik Goldschmidt GmbH | ARACHIDYL GLUCOSIDE DIETHYLHEXYL CARBONATE | 2.00 |
| Cetiol A | BASF AG | HEXYL LAURATE | 5.00 |
| | | | |

| Phase B | | | |
|---|---|---|---|
| RonaCare® Ectoin | Merck KGaA | ECTOIN | 0.50 |
| Pigment of Example 1 | | ALUMINA | 2.00 |
| Glycerol 85% | Merck KGaA | GLYCERIN, AQUA | 5.00 |
| Water, demineralized | | AQUA | |

| Phase C | | | |
|---|---|---|---|
| RonaFlair® Boroneige® SF-6 | Merck KGaA | BORON NITRIDE | 3.00 |
| Sepigel 305 | Seppic GmbH | LAURETH-7, POLYACRYLAMIDE, C13-14 ISOPARAFFIN | 2.00 |

| Phase D | | | |
|---|---|---|---|
| RonaCare® Luremin™ | Merck KGaA | SORBITOL, DIHYDROXY METHYLCHROMONE | 0.50 |
| Water, demineralized | | AQUA | 1.00 |

| Phase E | | | |
|---|---|---|---|
| Preservatives (q.s.) | | PARFUM | 0.00 |
| Fragrance (q.s.) | | | 0.00 |
| Citric acid solution 10% (q.s.) | | AQUA, CITRIC ACID | 0.00 |

Heat phases A and B separately to 80 °C. Add phase A and B while stirring. Homogenize. At 60 °C add the ingredients of phase C in the given order while strong mixing. Homogenize. Disperse RonaCare® Luremin™ in the water of phase D and add to the batch while stirring. Finally add preservative sand fragrance below 40 °C. Check pH and adjust to 5 - 5.5 with citric acid solution if necessary. Cool down to room temperature.

## Claims

1. α-Al₂O₃ flakes having a D₅₀-value of 5-15 µm and a D₈₀-value of less than 20 µm and a particle thickness of ≦ 500 nm prepared by a process consisting of the following steps:
(1) preparation of an aqueous solution of at least one water-soluble and/or insoluble aluminum salt which contains at least one sulfate compound,
(2) adding a basic solution selected from ammonia, sodium hydroxide, potassium hydroxide, sodium carbonate, potassium carbonate and combinations thereof and optionally at least one dopant to the aluminum salt solution (1), wherein total molar ratio of the sulfate compound to Al₂O₃ is 1 to ≦ 3.5 after step (2),
(3) drying of the obtained gel, followed by calcination, wherein the calcination temperature is from 900 to 1400 °C to obtain Al₂O₃ flakes and alkali salts in a molten salt,
(4) removal of the water soluble parts of the calcined molten salt obtained in step (3).

2. α-Al₂O₃ flakes according to Claim 1, wherein the sulfate compound is alkali metal sulfate.

3. α-Al₂O₃ flakes according to Claim 1 or 2, wherein the sulfate compound is selected from sodium sulfate, potassium sulfate, lithium sulfate and combinations thereof.

4. α-Al₂O₃ flakes according to one or more of Claims 1 to 3, wherein the dopant is selected from TiO₂, ZrO₂, SiO₂, SnO₂, In₂O₃, ZnO and combinations thereof.

5. α-Al₂O₃ flakes according to one or more of Claims 1 to 4, wherein the amount of the dopant is 0.01 - 5 % by weight based on the Al₂O₃ flake.

6. α-Al₂O₃ flakes according to one or more of Claims 1 to 5, wherein the dopant is TiO₂, SnO₂ or ZnO.

7. α-Al₂O₃ flakes according to one or more of Claims 1 to 6, **characterised in that** the MIU value (MIU value measured by the KES friction tester) is less than 0.8.

8. Use of the α-Al₂O₃ flakes according to one or more of Claims 1 to 7 as filler, as substrate for effect pigments, in formulations selected from paints, coatings, automobile coatings, automotive finishing, industrial coatings, powder coatings, printing inks, security printing inks, plastics, ceramic materials, cosmetics, glasses, paper, paper coating, toners for electrophotographic printing processes, seeds, greenhouse sheeting and tarpaulins, thermally conductive, self-supporting, electrically insulating, flexible sheets for the insulation of machines or devices, as absorber in the laser marking of paper and plastics, as absorber in the laser welding of plastics, pigment pastes with water, organic and/or aqueous solvents, in pigment preparations and dry preparations.

9. Use of the α-Al₂O₃ flakes according to Claim 8 as filler for cosmetic formulations.

10. Use of the α-Al₂O₃ flakes according to Claim 8 or 9 as filler for decorative application and personal care applications.

11. Formulation containing α-Al₂O₃ flakes according to one or more of Claims 1 to 7 in amounts of 0.01 - 95 % by weight, based on the formulation as a whole.

12. Formulation containing α-Al₂O₃ flakes according to Claim 11, **characterised in that** it contains at least one component selected from the group of water, polyols, polar and non-polar oils, fats, waxes, film formers, polymers, copolymers, surfactants, free-radical scavengers, antioxidants, stabilisers, odour enhancers, silicone oils, emulsifiers, solvents, preservatives, thickeners, rheological additives, fragrances, colorants, effect pigments, UV absorbers, surface-active assistants and/or cosmetic active compounds, fillers, binders, pearlescent pigments, color pigments and organic dyes.

## Patentansprüche

1. α-Al₂O₃-Plättchen mit einem D₅₀-Wert von 5-15 µm und einem D₈₀-Wert von weniger als 20 µm und einer Teilchendicke von ≦ 500 nm, hergestellt durch ein Verfahren, das aus den folgenden Schritten besteht:
(1) Herstellung einer wässrigen Lösung mindestens eines wasserlöslichen und/oder unlöslichen Aluminiumsalzes, die mindestens eine Sulfatverbindung enthält,
(2) Zugabe einer aus Ammoniak, Natriumhydroxid, Kaliumhydroxid, Natriumcarbonat, Kaliumcarbonat und Kombinationen davon ausgewählten basischen Lösung und gegebenenfalls mindestens eines Dotierstoffs zu der Aluminiumsalzlösung (1), wobei das Gesamtmolverhältnis der Sulfatverbindung zu Al₂O₃ nach Schritt (2) bei 1 bis ≦ 3,5 liegt,
(3) Trocknen des erhaltenen Gels, gefolgt von Kalzinierung, wobei die Kalzinierungstemperatur bei 900 bis 1400 °C liegt, so dass man Al₂O₃-Plättchen und Alkalisalze in einer Salzschmelze erhält,
(4) Entfernung der wasserlöslichen Anteile aus der in Schritt (3) erhaltenen kalzinierten Salzschmelze.

2. α-Al₂O₃-Plättchen nach Anspruch 1, wobei es sich bei der Sulfatverbindung um Alkalimetallsulfat handelt.

3. α-Al₂O₃-Plättchen nach Anspruch 1 oder 2, wobei die Sulfatverbindung aus Natriumsulfat, Kaliumsulfat, Lithiumsulfat und Kombinationen davon ausgewählt ist.

4. α-Al₂O₃-Plättchen nach einem oder mehreren der Ansprüche 1 bis 3, wobei der Dotierstoff aus TiO₂, ZrO₂, SiO₂, SnO₂, In₂O₃, ZnO und Kombinationen davon ausgewählt ist.

5. α-Al₂O₃-Plättchen nach einem oder mehreren der Ansprüche 1 bis 4, wobei die Menge des Dotierstoffs 0,01 - 5 Gew.-%, bezogen auf das Al₂O₃-Plättchen, beträgt.

6. α-Al₂O₃-Plättchen nach einem oder mehreren der Ansprüche 1 bis 5, wobei es sich bei dem Dotierstoff um TiO₂, SnO₂ oder ZnO handelt.

7. α-Al₂O₃-Plättchen nach einem oder mehreren der Ansprüche 1 bis 6, **dadurch gekennzeichnet, dass** der MIU-Wert (MIU-Wert gemessen mit dem KES-Reibungstester) weniger als 0,8 beträgt.

8. Verwendung der α-Al₂O₃-Plättchen nach einem oder mehreren der Ansprüche 1 bis 7 als Füllstoff, als Substrat für Effektpigmente, in Formulierungen, die ausgewählt sind aus Lacken, Farben, Autolacken, Autoreparaturlackierung, Industrielacken, Anstrichmittel, Pulverlacken, Druckfarben, Sicherheitsdruckfarben, Kunststoffen, keramischen Materialien, Kosmetika, Gläsern, Papier, Papierbeschichtung, Tonern für elektrophotographische Druckverfahren, Saatgut, Gewächshausfolien und Zeltplanen, wärmeleitenden, selbsttragenden, elektrisch isolierenden, flexiblen Folien zur Isolierung von Maschinen oder Vorrichtungen, als Absorber bei der Lasermarkierung von Papier und Kunststoffen, als Absorber beim Laserschweißen von Kunststoffen, Pigmentanteigungen mit Wasser, organischen und/oder wässrigen Lösungsmitteln, in Pigmentpräparationen und Trockenpräparaten.

9. Verwendung der α-Al₂O₃-Plättchen nach Anspruch 8 als Füllstoff für kosmetische Formulierungen.

10. Verwendung der α-Al₂O₃-Plättchen nach Anspruch 8 oder 9 als Füllstoff für dekorative Anwendung und Körperpflegeanwendungen.

11. Formulierung enthaltend α-Al₂O₃-Plättchen nach einem oder mehreren der Ansprüche 1 bis 7 in Mengen von 0,01-95 Gew.-%, bezogen auf die Gesamtformulierung.

12. Formulierung enthaltend α-Al₂O₃-Plättchen nach Anspruch 11, **dadurch gekennzeichnet, dass** sie mindestens eine Komponente enthält, die aus der Gruppe Wasser, Polyole, polare und unpolare Öle, Fette, Wachse, Filmbildner, Polymere, Copolymere, Tenside, Radikalfänger, Antioxidantien, Stabilisatoren, Geruchsverstärker, Silikonöle, Emulgatoren, Lösungsmittel, Konservierungsmittel, Verdicker, rheologische Zusatzstoffe, Duftstoffe, Farbmittel, Effektpigmente, UV-Absorber, oberflächenaktive Hilfsmittel und/oder kosmetische Wirkstoffe, Füllstoffe, Bindemittel, Perlglanzpigmente, Farbpigmente und organische Farbstoffe ausgewählt ist.

## Revendications

1. Paillettes d'α-Al₂O₃ ayant une valeur D₅₀ de 5-15 µm et une valeur D₈₀ inférieure à 20 µm et une épaisseur de particule ≦ 500 nm, préparées par un procédé constitué par les étapes suivantes :
(1) la préparation d'une solution aqueuse d'au moins un sel d'aluminium soluble et/ou insoluble dans l'eau qui contient au moins un composé de sulfate,
(2) l'addition d'une solution basique choisie parmi l'ammoniaque, l'hydroxyde de sodium, l'hydroxyde de potassium, le carbonate de sodium, le carbonate de potassium et des combinaisons de ceux-ci et éventuellement au moins un dopant à la solution de sel d'aluminium (1), où le rapport molaire total du composé de sulfate à l'Al₂O₃ va de 1 à ≦ 3,5 après l'étape (2),
(3) le séchage du gel obtenu, suivi d'une calcination, où la température de calcination va de 900 à 1400°C, afin d'obtenir des paillettes d'Al₂O₃ et des sels alcalins dans un sel fondu,
(4) l'élimination des parties solubles dans l'eau du sel fondu calciné obtenu dans l'étape (3).

2. Paillettes d'α-Al₂O₃ selon la revendication 1, dans lesquelles le composé de sulfate est un sulfate de métal alcalin.

3. Paillettes d'α-Al₂O₃ selon la revendication 1 ou 2, dans lesquelles le composé de sulfate est choisi parmi le sulfate de sodium, le sulfate de potassium, le sulfate de lithium et des combinaisons de ceux-ci.

4. Paillettes d'α-Al₂O₃ selon l'une ou plusieurs parmi les revendications 1 à 3, dans lesquelles le dopant est choisi parmi TiO₂, ZrO₂, SiO₂, SnO₂, In₂O₃, ZnO et des combinaisons de ceux-ci.

5. Paillettes d'α-Al₂O₃ selon l'une ou plusieurs parmi les revendications 1 à 4, dans lesquelles la quantité de dopant est de 0,01 - 5% en poids sur la base de la paillette d'Al₂O₃.

6. Paillettes d'α-Al₂O₃ selon l'une ou plusieurs parmi les revendications 1 à 5, dans lesquelles le dopant est TiO₂, SnO₂ ou ZnO.

7. Paillettes d'α-Al₂O₃ selon l'une ou plusieurs parmi les revendications 1 à 6, **caractérisées en ce que** la valeur MIU (valeur MIU mesurée par le testeur de friction KES) est inférieure à 0,8.

8. Utilisation des paillettes d'α-Al₂O₃ selon l'une ou plusieurs parmi les revendications 1 à 7, comme charge, comme substrat pour des pigments à effet, dans des formulations choisies parmi les peintures, les revêtements, les revêtements pour automobiles, les peintures pour la réparation automobile, les revêtements industriels, les peintures, les revêtements en poudre, les encres d'impression, les encres d'impression de sécurité, les matières plastiques, les matériaux céramiques, les cosmétiques, les verres, le papier, les revêtements de papier, les toners pour les procédés d'impression électrophotographique, les semences, les bâches et toiles pour serres, les feuilles flexibles thermiquement conductrices, autoporteuses, électriquement isolantes, pour l'isolement de machines ou de dispositifs, comme absorbant dans le marquage au laser de papier et de matières plastiques, comme absorbant dans le soudage au laser de matières plastiques, les pâtes de pigment avec de l'eau, des solvants organiques et/ou aqueux, dans des préparations de pigment et des préparations sèches.

9. Utilisation des paillettes d'α-Al₂O₃ selon la revendication 8, comme charge pour des formulations cosmétiques.

10. Utilisation des paillettes d'α-Al₂O₃ selon la revendication 8 ou 9, comme charge pour des applications décoratives et des applications de soin personnel.

11. Formulation contenant des paillettes d'α-Al₂O₃ selon l'une ou plusieurs parmi les revendications 1 à 7, selon des quantités de 0,01 - 95% en poids, sur la base de la formulation dans son ensemble.

12. Formulation contenant des paillettes d'α-Al₂O₃ selon la revendication 11, **caractérisée en ce qu'**elle contient au moins un composant choisi dans le groupe constitué par l'eau, les polyols, les huiles polaires et non polaires, les matières grasses, les cires, les agents filmogènes, les polymères, les copolymères, les agents tensioactifs, les agents antiradicalaires, les antioxydants, les stabilisants, les exhausteurs d'odeur, les huiles de silicone, les émulsifiants, les solvants, les conservateurs, les épaississants, les additifs rhéologiques, les fragrances, les colorants, les pigments à effet, les agents anti-UV, les auxiliaires tensioactifs et/ou les composés actifs cosmétiques, les charges, les liants, les pigments nacrés, les pigments colorés et les teintures organiques.
